# EUROPEAN PATENT SPECIFICATION

(11) **EP 2 913 057 B1**
(45) Date of publication and mention of the grant of the patent: **06.09.2017**
(21) Application number: 14185102.2
(22) Date of filing: 24.07.2009
(51) Int. Cl.: A61K 38/08, C07K 14/47

(54) **Cancer peptide therapeutics**
Krebspeptidtherapeutika
Thérapies peptidiques du cancer

(30) Priority: 24.07.2008 US 83393 P
(43) Date of publication of application: 02.09.2015
(62) Divisional of application: 09790794.3
(73) Proprietor: Indiana University Research and Technology Corporation, Indianapolis, IN 46202 (US)
(72) Inventor: Hickey, Robert J., Indianapolis, IN 46202 (US); Malkas, Linda H., Indianapolis, IN 46202 (US)
(74) Representative: Oficina Ponti, SLP

(56) References cited:
- EP-A1- 1 586 652
- WO-A2-2007/098415
- WO-A2-2010/011890

## Description

### TECHNICAL FIELD

This disclosure relates to peptide-based therapeutic compositions and methods to inhibit cancer cell proliferation.

### BACKGROUND

Proliferating cell nuclear antigen (PCNA) plays an important role in the process of DNA replication, repair, chromosomal recombination, cell cycle check-point control and other cellular proliferative activities. In conjunction with an adaptor protein, replication factor C (RFC), PCNA forms a moving clamp that is the docking point for DNA polymerases delta and epsilon. Different isoforms of proliferating cell nuclear antigen (PCNA) that display both acidic and basic isoelectric points (pl) have been demonstrated. Analysis of PCNA by two-dimensional polyacrylamide gel electrophoresis (2D PAGE) from both malignant and non-malignant breast cells (referred to as non-malignant PCNA or nmPCNA) and tissues revealed the presence of an acidic form of PCNA only in malignant cells (referred to as the cancer-specific PCNA or csPCNA or caPCNA). This difference in isoelectric point between these two forms of PCNA, appears to result from an alteration in the ability of the malignant cells, to post-translationally modify the PCNA polypeptide and is not due to a genetic change within the PCNA gene.

Structural work examining the structure of the PCNA polypeptide to define the structural differences between the caPCNA and non-malignant cell isoform of PCNA revealed a region of the caPCNA protein that is uniquely exposed only in the cancer cell. An antibody was developed to a region of the cancer specific isoform of PCNA that is highly selective for the PCNA isoform expressed exclusively in cancer cells.

Proliferating cell nuclear antigen (PCNA) is a 29 kDa nuclear protein and its expression in cells during the S and G2 phases of the cell cycle, makes the protein a good cell proliferation marker. It has also been shown to partner in many of the molecular pathways responsible for the life and death of the cell. Its periodic appearance in S phase nuclei suggested an involvement in DNA replication. PCNA was later identified as a DNA polymerase accessory factor in mammalian cells and an essential factor for SV40 DNA replication in vitro. In addition to functioning as a DNA sliding clamp protein and a DNA polymerase accessory factor in mammalian cells, PCNA interacts with a number of other proteins involved in transcription, cell cycle checkpoints, chromatin remodeling, recombination, apoptosis, and other forms of DNA repair. Besides being diverse in action, PCNA's many binding partners are linked by their contributions to the precise inheritance of cellular functions by each new generation of cells. PCNA may act as a master molecule that coordinates chromosome processing.

PCNA is also known to interact with other factors like FEN-1, DNA ligase, and DNA methyl transferase. Addi-tionally, PCNA was also shown to be an essential player in multiple DNA repair pathways. Interactions with proteins like the mismatch recognition protein, Msh2, and the nucleotide excision repair endonuclease, XPG, have implicated PCNA in processes distinct from DNA synthesis. Interactions with multiple partners generally rely on mechanisms that enable PCNA to selectively interact in an ordered and energetically favorable way.

The use of short synthetic peptides for the generation of polyclonal and monoclonal antibodies has been used with considerable success. Peptides are known to serve as chemo-attractants, potent neurological and respiratory toxins, and hormones. The peptides have also been used as affinity targets and probes for biochemical studies, and have provided a basis for understanding the characteristics and specific nature of discrete protein-protein interactions. In addition, peptide hormones exert potent physiological effects, and in some cases the active hormone is either a peptide that is contained within a larger protein or is processed and released from a precursor protein prior to exerting its physiological effect.

Peptides have been used to disrupt protein-protein interactions, by acting as highly specific competitors of these interactions. Biochemical studies employing peptide reagents advanced the use of peptides as therapeutic drugs capable of disrupting cell functions that require protein-protein interactions. Thus, specific cellular processes such as apoptosis and cell cycle progression, which are dependent upon discrete protein-protein interactions, can be inhibited if these protein-protein interactions are selectively disrupted. The replication of genomic DNA being dependent on protein-protein interactions is also susceptible to peptide-induced inhibition of these protein interactions.

*In vivo* DNA synthesis is a highly regulated process that depends on a myriad of biochemical reactions mediated by a complex series of protein-protein interactions. Cell division is dependent on the DNA synthetic process, and cancer cell growth is substantially sensitive to any agent that disrupts the regulation and/or the activity of the DNA synthetic machinery responsible for copying the cancer cell's genomic DNA. In addition, it was demonstrated that one signature of breast cancer is the induction of genomic instability, as transformed cells develop a highly aggressive metastatic phenotype. Genomic instability arises through a series of changes in the cellular DNA synthetic machinery that alters the fidelity with which DNA is synthesized.

Studies utilizing the carboxyl terminal 26 amino acids from the p21 cip protein, (which is known to interact with the PCNA protein), demonstrated the ability of this peptide to disrupt the cellular proliferative process. This peptide fragment of p21 potentially disrupts one or more cellular processes utilizing PCNA and presumably interferes with critical protein-protein interactions that participate in the DNA synthetic process as well as the regulation of other cell cycle check-point controls and the induction of apoptosis.

Studies utilizing this peptide fragment of p21 have demonstrated the ability of the p21 peptide to activate a non-caspase associated apoptotic pathway. Similarly, studies involving a 39 amino acid peptide fragment of the p21 protein partially inhibited DNA replication *in vivo,* and suggest that this peptide fragment of p21 can stabilize the PCNA-p21 protein interaction leading to the decrease in DNA synthetic activity within the cell.

In addition, computational chemical methods are being used to model specific regions of the PCNA molecule that may interact with other cellular proteins involved in cell cycle check point control and DNA synthesis. Regions of the cyclin-CDK complex may serve as templates to identify target sites for disrupting key cell cycle check-point control points that are essential for cell proliferation.

Use of synthetic peptides to inhibit cell proliferation and the process of selectively targeting cancer specific PCNA protein to mediate the inhibition of cell proliferation is needed to treat cancer. Peptidomimetic drugs that interact with an antigenic site or target site on caPCNA to disrupt specific protein-caPCNA interactions that are unique to the cancer cell are desired. Peptides derived from caPCNA specific epitopes, disclosed herein, significantly augment the cytotoxic effects of specific traditional chemotherapeutic regimens and consequently kill cancer cells in a highly selective manner.

Document EP1586652 provides recombinant DNA molecules for expression of a protein, and recombinant DNA molecules for expression of mRNA complementary to at least a portion of an mRNA native to the target plant for use in gene suppression to suppress the expression of a protein. In particular, it discloses a protein according to the sequence SEQ ID NO: 5273. No therapeutic use for that protein is mentioned.

Document WO2007/098415 disclose peptides derived from cancer specific isoform of proliferating cell nuclear antigen (caPCNA, also known as csPCNA) or from nmPCNA-interacting proteins which interfere with intracellular protein-protein interaction, thereby causing a reduction in the proliferative potential of cancer. These peptides serve as therapeutic compositions to reduce the proliferation of cancer cells and also augment existing chemotherapeutic methods.

### SUMMARY

The present invention relates to a therapeutic composition for use according to the set of claims.

Peptide variants derived from specific regions or domains of cancer specific (caPCNA)-interacting proteins interfere with the interaction of cellular proteins with the PCNA *protein in vivo* and thereby affect cancer cell survival. Specific amino acid sequences representing peptide fragments of the caPCNA protein disrupt the regulatory activity of PCNA and subsequently inhibit cancer cell growth through the disruption of functioning of cellular processes that require PCNA, including DNA replication, repair, chromosomal recombination, and cell cycle check-point control.

Amino acid substitutions in one or more positions improve the cytotoxic effects of caPCNA-derived peptides. caPCNA-derived peptides including amino acid substituted peptides that have tags or domains that enhance cellular uptake, increase the cytotoxic effects of the caPCNA peptides.

A therapeutic composition for reducing cellular proliferation of malignant cells includes a cell permeable peptide molecule having an amino acid sequence selected from the group consisting of LGIPEQEY, LAIPEQEY, LGIAEQEY, LGIPAQEY, LGIPEAEY, LGIPEQAY, LGIAEAEY, LGIPEAAY, LGIAEQAY, and LGIAEAAY. In an embodiment, the peptide molecule is a synthetic molecule.

A therapeutic composition for reducing cellular proliferation of malignant cells includes a cell permeable peptide molecule consisting essentially of an amino acid sequence selected from the group consisting of LGIPEQEY, LAIPEQEY, LGIAEQEY, LGIPAQEY, LGIPEAEY, LGIPEQAY, LGIAEAEY, LGIPEAAY, LGIAEQAY, and LGIAEAAY.

In an embodiment, the caPCNA-derived peptide molecules further include a cell permeable factor or a cell-uptake agent. For example, the cell permeable factor is a cell penetrating peptide selected from the group of amino acid sequences RRRRRRR, RRRRRRRR, RRRRRRRRR, RRRRRRRRRR, RRRRRRRRRRR, RQIKIWFQNRRMKWKK, GRKKRRQRRRPPQ, GWTLNSAGYLLGKINLKALAALAKKIL, GRKKRRQRRR or a factor listed on Table 4. In some aspects, the cell penetrating peptide is covalently linked or conjugated to the peptide molecules derived from caPCNA. In other aspects, the cell penetrating peptide is recombinantly fused with the peptide molecule. In certain embodiments, the cell penetrating peptide further includes a spacer sequence. The spacer sequence may be about 1-10 or about 1-20 amino acids in length. Synthetic spacers are also suitable as long as they do not interfere with the translocation of the peptide across the cell membrane.

In an embodiment, the peptide is engineered to be protease resistant compared to an unmodified native caPC-NA-derived peptide. In an embodiment, the peptides disclosed herein are generated as retro-inverso isomers.

In an embodiment, the composition that includes a caPCNA-derived translocatable peptide may further include a chemotherapeutic agent. Preferably, the chemotherapeutic agent is a DNA damaging agent. Examples of DNA dam-aging agents include for example, doxorubicin, irinotecan, cyclophosphamide, chlorambucil, melphalan, methotrexate, cytarabine, fludarabine, 6-mercaptopurine, 5- fluorouracil, capecytabine, cisplatin, carboplatin, oxaliplatin, and a com-bination thereof.

In an embodiment, the compositions that include the peptide inhibitors of caPCNA interaction may be delivered as a liposome. The liposome may also include nanoparticles. In an aspect, one or more constituents of the pharmaceutical composition that includes caPCNA-derived peptides may further include a nanoparticle.

A method to inhibit the growth of a cancer cell, the method includes:
(a) obtaining a therapeutically effective amount of the composition that includes a peptide disclosed herein;
(b) administering the composition such that the agent contacts a population of cancer cells; and
(c) inhibiting the growth of the cancer cell with the composition.

In an embodiment, the composition is administered intravenously. Any mode of administration, including direct delivery to cancer is possible. A chemotherapeutic agent may be administered to a cancer patient prior to or along with or after the administration of the composition that includes the caPCNA-derived peptides or variants thereof. In an embodiment, radiotherapy may also be administered prior to or along with or after the administration of the composition that includes the caPCNA-derived peptides or variants thereof. Radio therapy includes beam radiation therapy and radio isotope therapy.

A method of sensitizing cancer cells for cancer therapy includes:
(a) administering a therapeutically effective amount of the composition that includes a peptide molecule comprising an amino acid sequence selected from the group of LGIPEQEY, LAIPEQEY, LGIAEQEY, LGIPAQEY, LGIPEAEY, LGIPEQAY, LGIAEAEY, LGIPEAAY, LGIAEQAY, and LGIAEAAY, such that the composition contacts a population of cancer cells to sensitize the cancer cells; and
(b) administering a chemotherapeutic agent or radiotherapy to inhibit the growth of the cancer cells.

A method of chemoprevention of cancer includes:
(a) administering a chemopreventative agent comprising the composition of composition that includes a peptide molecule comprising an amino acid sequence selected from the group of LGIPEQEY, LAIPEQEY, LGIAEQEY, LGIPAQEY, LGIPEAEY, LGIPEQAY, LGIAEAEY, LGIPEAAY, LGIAEQAY, and LGIAEAAY, such that the compo-sition contacts a population of cells that are likely to express cancer specific isoform of PCNA (caPCNA); and
(b) preventing the transformation of the cells that express the caPCNA isoform to a malignant state or develop tumors.

In an embodiment, a chemopreventative agent is administered to an individual considered high-risk for a cancer type. In an embodiment, the chemopreventative agent is administered to an individual with pre-cancerous cells.

Rational drug design methodologies can also be implemented to obtain specific inhibitors of caPCNA cellular interaction based on the structural or sequence information of a caPCNA derived peptide, e.g., a peptide that has an amino acid sequence LGIPEQEY. In an embodiment, the agent is a peptide fragment derived from an intracellular protein. In an embodiment, the intracellular protein is known to interact with caPCNA.

A therapeutic composition for reducing in vivo cellular proliferation of malignant cells that express a cancer specific isoform of proliferating cell nuclear antigen (caPCNA), the composition includes a peptide molecule that has an amino acid sequence LGIPEQEY with a cell-permeable peptide sequence comprising a polyarginine sequence. In an embodiment, the peptide domain that facilitates peptide uptake across cells is R9 (nonarginine tag).

A liposome composition for reducing in vivo cellular proliferation of malignant cells that express a cancer specific isoform of proliferating cell nuclear antigen (caPCNA), the composition includes a peptide molecule comprising an amino acid sequence R9-LGIPEQEY with one or more amino acid substitutions or a functionally equivalent structure thereof or a peptidomimetic thereof, wherein R9 is either conjugated chemically or is part of a recombinant fusion protein.

A suitable cell surface targeting factor that is used along with one or more of the compositions disclosed herein is selected from the group of HER2/neu, estrogen receptor, progesterone receptor, epidermal growth factor receptor (EGFR).

A recombinant cell that expresses a caPCNA-derived peptide, wherein the peptide selectively disrupts protein-protein interaction in cancer cells. In an embodiment, the caPCNA-derived peptide includes an amino acid sequence LGIPEQEY with one or more amino acid substitutions.

A synthetic peptide that includes an amino acid sequence LGIPEQEY with one or more amino acid substitutions and a peptide translocation sequence.

Other suitable PCNA-derived peptide inhibitors wherein one or more amino acids are substituted include QL-GIPEQEYSC, VEQLGIPEQEY, LGIPEQEYSCVVK, LGIPEQEYSCVVKMPSG, EQLGIPEQEY, QLGIPEQEY, LGIPEQEYSCVVKMPS, LGIPEQEYSCVVKMP, LGIPEQEYSCVVKM, LGIPEQEYSCVV, LGIPEQEYSCV, LGIPEQEYSC, QLGIPEQEYSC, LGIPEQEYS that have one or more amino acid substitutions and combinations of the additional NH₂ and COOH termini amino acids that flank LGIPEQEY with one or more amino acid substitutions and a cell-permeable sequence such as R9.

Replication defective viral expression vectors (e.g., lentivirus, adenovirus, adeno-associated virus, herpes virus and others) capable of expressing the peptides disclosed herein are also suitable delivery systems.

### BRIEF DESCRIPTION OF THE DRAWINGS

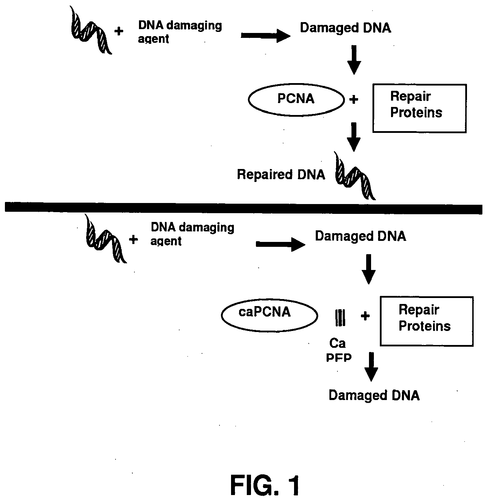

**FIG. 1** illustrates the likely mode of action of caPCNA-based peptide inhibitors and their roles in inhibiting cancer proliferation in the presence of a DNA damaging agent.

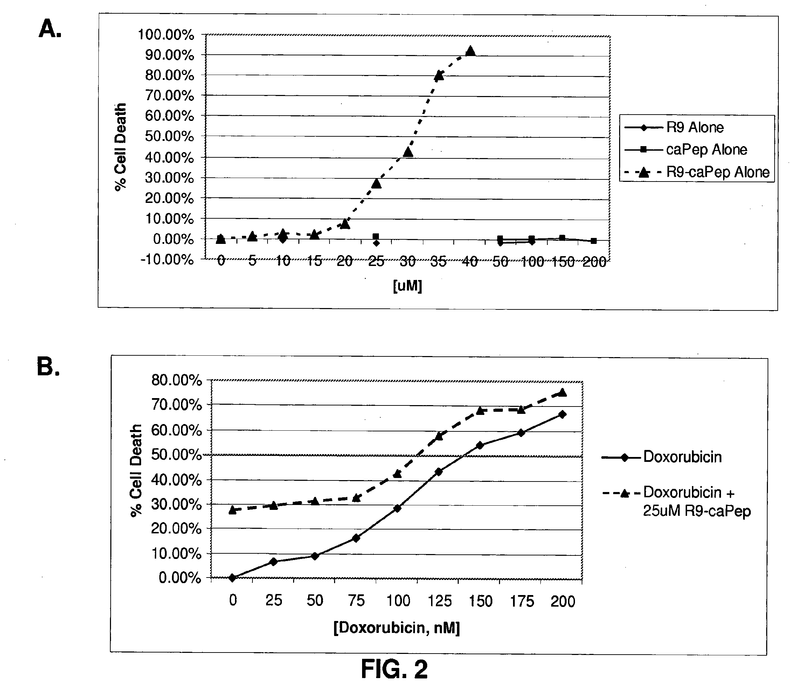

**FIG. 2** shows **(A)** cytotoxic effects of R9, caPeptide (126-133), and R9-caPep. U937 cells were treated with increasing amounts of each peptide and the level of apoptosis was evaluated by flow cytometry; and **(B)** Combined cytotoxicity of doxorubicin and R9-caPep. U937 cells were treated with 25mM R9-caPep and increasing concentrations of doxorubicin. The IC50 (thicker line) of doxorubicin decreases from 140nM to 110nM when 25mM of R9-caPep is added.

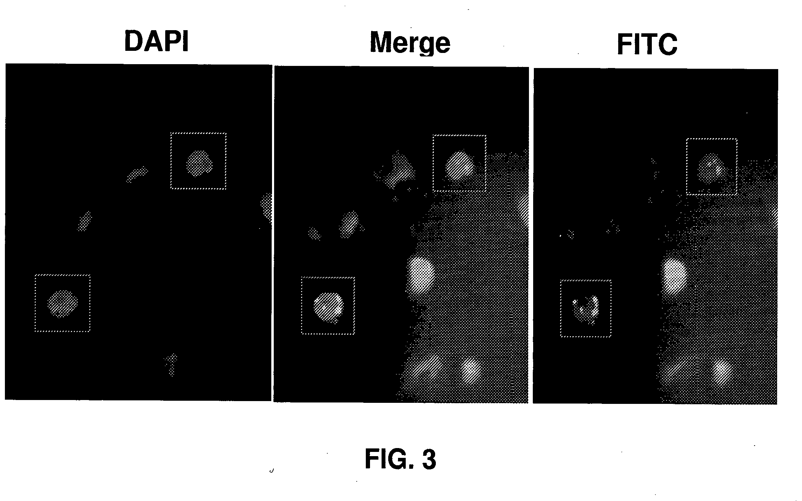

**FIG. 3** shows nuclear localization of R9-caPep. U937 cells were treated with FITC-R9-caPep and collected on coated slides by cytospin. Fluorescent images for the DAPI stained nuclei (left) and FITC-R9-caPep (right) were overlaid (center) showing cellular uptake and nuclear localization of R9-caPep. Boxes indicate intact cells.

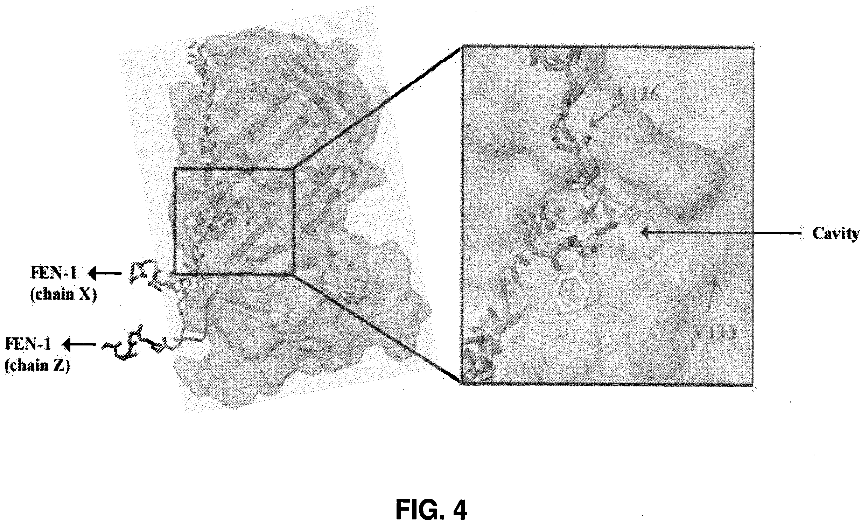

**FIG. 4** shows structural interactions of FEN-1 with PCNA. Left: PCNA monomers A and C with the molecular surface shown. aa330-380 of FEN-1 monomers X and Z shown in wireframe representation. Right: Close-up of the region of strong hydrophobic interactions conserved in the PCNA/FEN-1 interaction. aa126 and aa133 form the edge of the cavity where FEN-1 interacts with PCNA.

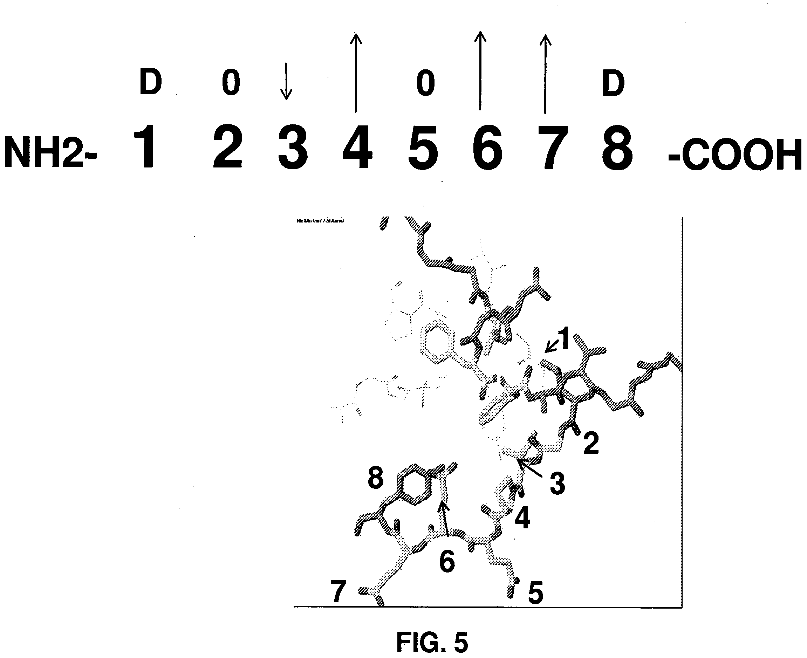

**FIG. 5** shows the interactions of aa126-133 peptide in the binding pocket.

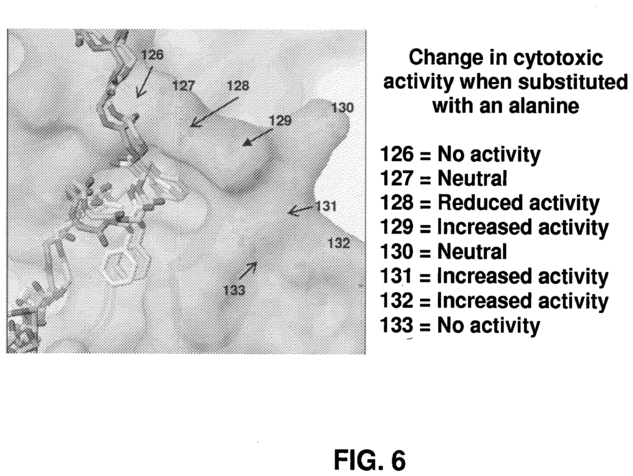

**FIG. 6** shows orientation of amino acids 126-133 in the pocket surrounding FEN-1. Portion of FEN-1 X and Z chains that fit into the cavity created by the molecular surface representation of PCNA. Position of each amino acid from PCNA peptide 126-133 is indicated. Chart to the right lists the change in cytotoxicity when each amino acid is substituted with an alanine.

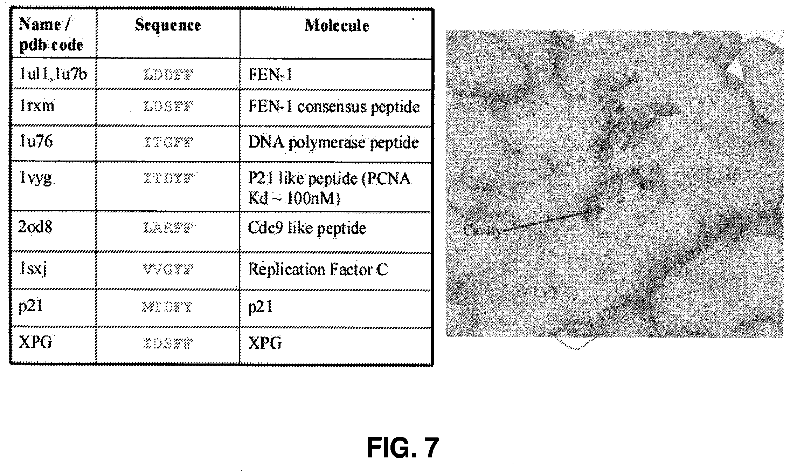

**FIG. 7** illustrates conserved residues of binding partners for PCNA. Left: Sequence conservation for various pep-tides/proteins and the five amino acid sequence which binds in the cavity partially created by aa126-133 of PCNA. Right: Overlay of the conserved sequences of the PCNA binding partners shown in the cavity. The most conserved residues are indicated in both the table and the figure.

### DETAILED DESCRIPTION

The present invention relates to a therapeutic composition for use according to the set of claims.

Methods and compositions disclosed herein relate to caPCNA-peptide variants, peptidomimetics, functional analogs thereof that selectively disrupt vital cellular functions in cancer cells. There are at least two modes of actions of these peptides. For example, caPCNA-derived peptide variants either compete with caPCNA to bind to caPCNA-interacting proteins or alternatively bind to a site on caPCNA-interacting protein that disrupts the interaction.

Specific peptide variants derived from the caPCNA protein sequence have the ability to block the binding of several cellular proteins that participate in DNA replication, repair, cell cycle control, apoptosis, transcription, or chromosomal recombination in cancer cells. The binding of caPCNA to these cellular proteins is disrupted when the peptide is allowed to compete with these proteins for their naturally occurring binding site on PCNA. By disrupting the naturally occurring interaction between PCNA and the proteins that bind to or interact with PCNA, normal cellular functions that recruit PCNA are disrupted. This disruption of vital cellular machinery renders the caPCNA-derived peptide variants cytotoxic by themselves or in combination with other molecules, such as, for example cancer chemotherapeutic drugs. These peptides, either alone or in combination with other cancer therapy agents are useful cancer chemotherapeutics or augmentors of the pharmacodynamic effect of specific anti-cancer chemotherapeutics. The peptide inhibitors disclosed herein sensitize tumor cells towards chemotherapy agents that damage DNA and also render tumors that are generally resistant to cancer drugs more responsive.

The term "sensitize" as used herein, for therapeutic purposes, generally refers to the ability of the peptides disclosed herein to lower the amount of a growth inhibitory agent or a cytotoxic agent (e.g., doxorubicin) needed to kill 50% of a group of cancer cells (e.g., a tumor) within a defined period of time (e.g., 24, 48, 72 hours).

The terms "pre-cancerous" or "pre-malignant" generally refers to a condition which may or may not be recog-nizable as a morphological change in tissue architecture that is known to be, or thought to be, associated with the development of a cancer within that tissue (organ). In addition, the initial molecular changes in gene expression, (or expression of specific isoforms of proteins), known to be associated with some percentage of cancer cells that are found in tumor tissues, may precede readily discernable morphological changes within the cells of these tissues undergoing the cancer transformation process. Thus the initial changes in the expression patterns of specific genes and/or proteins may be the first events associated with the molecular transformation process leading to the development of a cancer; may only be recognizable at the molecular level, as they have not in themselves induced an alteration in the morphology of the cells, and/or tissue, to the extent that it can be recognized by a trained individual (pathologist) at the light microscopic level.

The term "augment" or "augmenting" as used herein, for therapeutic purposes, generally refers to an improve-ment in the pharmacodynamic effect (referred to as the efficacy) of a therapeutic agent. Thus, the term "augment" refers to the ability of the peptide to raise the efficacy of a therapeutic agent (e.g., doxorubicin) leading to the killing of a greater number of cancer cells over the same unit of time (e.g., 24, 48, or 72 hour period) when the peptide is administered along with the therapeutic agent as compared to the therapeutic agent alone.

Peptide variants derived from the protein Proliferating Cell Nuclear Antigen (PCNA) are identified herein that have the ability to act, in conjunction with DNA damaging agents (e.g., doxorubicin), to enhance the therapeutic effects of such agents to treat a variety of cancer cells. See FIG. 1 that illustrates the likely mode of action of caPCNA-based peptide inhibitors and their roles in inhibiting cancer proliferation in the presence of a DNA damaging agent. The peptides are derived from the amino acid sequence within PCNA, for example, encompassing amino acids 126-133 and include one or amino acid mutations.

caPCNA-derived peptide variants and peptidomimetics represent novel anti-cancer therapeutic agents and also augment or sensitize tumor cells towards existing cancer therapies.

The peptide sequences disclosed herein target a region of the caPCNA protein that is likely to be uniquely unfolded in cancer cells. Thus, the peptides disclosed herein are designed to selectively target tumor cells by virtue of their ability to compete with caPCNA for regulating the activity of specific proteins interacting with the amino acid sequences within PCNA that are involved in at least one of the following cellular processes: DNA replication, repair, recombination, transcription, cell cycle checkpoint control, and apoptosis.

The peptides disclosed herein are synthesized using standard peptide synthesis procedures and equipments or can be obtained commercially (e.g., United Biochemical Research Co., Seattle WA). A caPCNA-derived peptide that includes amino acids 126-133 of the human PCNA molecule (LGIPEQEY) having at least one amino acid substitution, followed by a cell penetrating peptide (CPP) sequence, e.g., a polyarginine sequence to facilitate uptake of the peptide into cells selectively inhibits cancer cells in vitro.

In certain embodiments involving in vitro methodologies, uptake of caPCNA peptide variants were initiated by incubation of this peptide with the cancer cells in the presence of dimethyl sulfoxide (DMSO) in either phosphate buffered saline (PBS) or culture media containing 0.2-2% DMSO, without serum for about 4-24 hours. Uptake of these peptides is also efficiently mediated by encapsulation of the peptide in a liposome formulation and subsequent incubation with the cancer cells at 37°C for about 4-24 hours. These peptides also augment the cytotoxic effects of chemotherapeutic agents such as doxorubicin.

The term "agent" as used herein includes nucleic acids, proteins, protein fragments, peptides, synthetic peptides, peptidomimetics, analogs thereof, small molecules, inhibitors, and any chemical, organic or bioorganic molecule capable of affecting protein-protein interaction or a cellular process.

The terms "caPCNA peptide variants" or "peptide variants" mean peptides whose sequences were derived from caPCNA and include one or more mutations such as substitution mutations or deletion mutations or amino acid analogs or a combination thereof. For example, LGIPEQEY representing amino acids 126-133 of PCNA is caPCNA derived sequence in which for example, amino acids G, P, Q, and penultimate E can be substituted with an amino acid such as alanine (A). Thus, LX₁IX₂EX₃X₄Y is a peptide variant wherein X₁₋₄ can be substituted either independently or collectively. In an embodiment, X₁ is A, X₂ is P, X₃ is Q, and X₄ is E. In another embodiment, X₁ is G, X₂ is A, X₃ is Q, and X₄ is E. In another embodiment, X₁ is G, X₂ is P, X₃ is A, and X₄ is E. In another embodiment, X₁ is G, X₂ is P, X₃ is Q, and X₄ is A. In another embodiment, X₁ is G, X₂ is A, X₃ is A, and X₄ is A. The "caPCNA peptide variants" or "peptide variants" can range from about 5-10, 5-50, 7-50, 8-20, 8-25, 8-30, 8-40, 8-50 amino acids in length. For example, the "caPCNA peptide variants" or "peptide variants" may consist essentially of about 5, 6, 7, 8, 9, 10, 11, 12, 13, 14, 15, 16, 17, 18, 19, 20, 25, 30, 35, 40, 45, or 50 contiguous amino acids from caPCNA wherein one or more amino acids are substituted. The "caPCNA peptide variants" or "peptide variants" can further peptide translocation domains or sequences that enable the "caPCNA peptide variants" or "peptide variants" to penetrate or translocate across cellular membranes. The "caPCNA peptide variants" or "peptide variants" are also be modified to affect their lipophilicity to enhance peptide delivery into cancer cells. The peptides can be synthesized ("synthetic peptides") or can also be produced through recombinant techniques ("recombinant peptide") or expressed *in vivo* using gene expression techniques. These peptides can also be engineered to increase their *in vivo* stability (e.g., increase peptide stability by rendering them protease resistant) without significantly affecting their efficacy in inhibiting caPCNA-protein interactions. Mutations including in-sertions, deletions, substitutions, amino acid modifications that substantially do not affect the inhibitory activity of the peptides disclosed herein are within the scope. Peptides that consist essentially of the 126-133 sequence LGIPEQEY having one or more mutations may include other heterologous sequences that do not materially affect the inhibitory function of the peptide variants disclosed herein.

The peptides disclosed herein show specificity to killing malignant cells compared to cancer cells. For example, the peptides disclosed herein are substantially specific in which the peptides preferentially kill malignant cancer cells more than 50%, preferably more than 60% or 70% or 80% or 90% or 95% when compared to non-malignant cells.

A "peptide variant" or "peptide derivative" also means a molecule having an amino acid sequence of a region that is similar to a portion of PCNA or of a PCNA homolog, but additionally having at least one chemical modification of one or more of its amino acid side groups, α-carbon atoms, terminal amino group, or terminal carboxylic acid group. A chemical modification includes adding chemical moieties, creating new bonds, and removing chemical moieties. Modi-fications at amino acid side groups include acylation of lysine, ε-amino groups, N-alkylation of arginine, histidine, or lysine, alkylation of glutamic or aspartic carboxylic acid groups, and deamidation of glutamine or asparagine. Modifications of the terminal amino include the des-amino, N-lower alkyl, N-di-lower alkyl, and N-acyl modifications. Modifications of the terminal carboxy group include the amide, lower alkyl amide, dialkyl amide, and lower alkyl ester modifications. A lower alkyl is a C1-C4 alkyl. Furthermore, one or more side groups, or terminal groups, may be protected by protective groups known to the ordinarily-skilled protein chemist. The α-carbon of an amino acid may be mono-or di-methylated.

Those of skill in the art recognize that peptides may be substantially similar to the peptides described above in that an amino acid residue may be substituted with another amino acid residue having a similar side chain without substantially affecting the inhibitory functions of the peptide variants disclosed herein. For example, a group of aminoacids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acid substitution groups include: valine-leucine-isoleucine, phenylalanine-tyrosine, lysine-arginine, alanine-valine, and asparagine-glutamine. Thus, the peptide inhibitors disclosed herein may have one or more conservative amino acid substitutions without substantially affecting the inhibitory functions of the peptide.

Non-naturally occurring variants of the caPCNA-derived peptides can readily be generated using recombinant techniques or chemically synthesized. Such variants include, but are not limited to deletions, additions and substitutions in the amino acid sequence of the disclosed peptides. For example, one class of substitutions is conserved amino acid substitution. Such substitutions are those that substitute a given amino acid in the disclosed caPCNA-derived peptides by another amino acid of like characteristics. Typically accepted as conservative substitutions are the replacements, one for another, among the aliphatic amino acids Ala, Val, Leu, and IIe; interchange of the hydroxyl residues Ser and Thr; exchange of the acidic residues Asp and Glu; substitution between the amide residues Asn and Gln; exchange of the basic residues Lys and Arg; and replacements among the aromatic residues Phe and Tyr. A list of possible amino acid substitutions is provided in Table 5 herein.

The term "variant" refers to a peptide having an amino acid sequence that differ to some extent from a native sequence peptide, that is an amino acid sequence that vary from the native sequence by conservative amino acid substitutions, whereby one or more amino acids are substituted by another with same characteristics and conformational roles. The amino acid sequence variants possess substitutions, deletions, and/or insertions at certain positions within the amino acid sequence of the native amino acid sequence.

The PCNA-derived peptide variants can also be fused or otherwise linked to a ligand for a cell surface receptor that is present in cancer cells. The ligand is optionally cleavable such that the peptide variants (inhibitors) are targeted to a tumor cells using a tumor-specific ligand but are cleaved by a protease present in the tumor environment such that the peptide variants are free to enter the tumor cells. For example, the human transferrin receptor (hTfR), a marker for cellular proliferation is used as a target for therapeutics and is expressed at least 100-fold more in oral, liver, pancreatic, prostate, and other cancers (Lee et al., (2001) "Receptor mediated uptake of peptides that bind the human transferrin receptor" Eur. J. Biochem., 268: 2004-2012). Peptides, HAIYPRH and THRPPMWSPVWP bind specifically hTfR and these peptides were able to target associated macromolecule to the hTfR (Lee, *supra*). These peptides bind sites that do not overlap with the native ligand, Tf, and are useful *in vivo* for targeting macromolecules to the endocytic pathway in hTfR-positive cells (Lee, *supra*). Such peptides can also be used to target PCNA-derived peptides to enhance peptide delivery and also to further enhance specific delivery.

The term "cell permeable factor" or "cell membrane carrier" or "cell penetrating element" refers to any component including peptides that enhance the ability of the peptide variants disclosed herein to translocate the cell membrane as long as the factor does not substantially affect the ability of the peptide variants to inhibit caPCNA interaction. Optionally, the cell-permeable factors operate through a non-endocytic and non-degradative pathway in mammalian cells. These factors may include cell penetrating peptides (CPP) or cell permeable peptides. Examples of suitable cell-permeable peptides or peptide domains to link or fuse caPCNA-derived peptides include, for example, small polybasic peptides derived from the transduction domains of certain proteins, such as polyarginine (R6-R21), the third - helix of the Antennapedia (Antp) homeodomain, an RYIRS tag sequence, and those listed in Table 4 herein.

In an embodiment, the cell membrane permeable carrier is a peptide, preferably an arginine rich peptide. (see e.g., Futaki S. et al., (2001) "Arginine-rich peptides. An abundant source of membrane-permeable peptides having potential as carriers for intracellular protein delivery" J. Biol. Chem., 276, 5836). The number of arginine residues in a cell membrane permeable carrier peptide may contain more than 6 arginines, preferably 7, 8, 9, 10, 11, 12, 13, 14, or 15 arginine residues. The arginine residues in an arginine rich peptide need not be contiguous. Those of skill in the art know how to select an appropriate arginine rich peptide with a suitable number of arginine residues.

A peptide hairpin can also be used to make use of the increased number of extracellular proteases surrounding tumor tissues (see US20070041904, incorporated herein by reference) to deliver the inhibitors ("cargo") disclosed herein. The construct includes a polyarginine peptide covalently attached to a polyanionic segment, which would only be sub-stantially internalized upon proteolytic cleavage of the anionic domain. Because the protease targeted is likely overexpressed on cancerous cells, internalization is more likely with the tumor cells compared to a normal cell. Cellular asso-ciation of cell-penetrating peptides (CPPs) (e.g., polyarginine based) is blocked when they are fused to an inhibitory domain made up of negatively charged residues. Such fusions termed as activatable CPPs (ACPPs) because cleavage of the linker between the polycationic and polyanionic domains, usually by a protease, releases the CPP portion and its attached peptide of interest ("cargo") to bind to and enter cells such as tumor cells.

Pretreatment of the cell with a polycation, cationic polymer and/or cationic peptide before transportation of the heterologous compound into the cell is also useful (see e.g., 20060083737, incorporated herein by reference).

Nuclear localization sequences (NLS) for example, VQRKRQKLMP, SKKKKIKV, and GRKRKKRT are also useful in transporting the peptide variants disclosed herein into tumor cells. Other NLS can be obtained for example at Nair et al., (2003), NLSdb: database of nuclear localization signals, Nucl. Acids Res., 31:397-399 (see also http://cubic.bioc.columbia.edu/db/NLSdb/).

In some embodiments, the peptide variants disclosed herein are conjugated to the cell membrane permeable carrier, optionally including a spacer. For example, a polyarginine peptide having 5-9 arginine residues may optionally include a non-arginine-based spacer peptide or a spacer having non-standard amino acids or amino acid analogs. The spacers generally provide additional length to minimize for example steric hindrance to the function or transport of the peptide variants disclosed herein. Spacers may include about 1, 2, 3, 4, 5, 6, 7, 8, 9, 10, 15 or more amino acids. Suitable amino acids for use as spacers include for example, glycine

The caPCNA peptide variants and the cell-permeable peptides are linked by chemical coupling in any suitable manner known in the art as long as rendering the conjugated proteins are biologically active. In an aspect, one way to increase coupling specificity is to directly chemical coupling to a functional group found only once or a few times in one or both of the polypeptides to be cross-linked. For example, in many proteins, cysteine, which is the only protein aminoacid containing a thiol group, occurs only a few times. Also, for example, if a polypeptide contains no lysine residues, a cross-linking reagent specific for primary amines will be selective for the amino terminus of that polypeptide. Successful utilization of this approach to increase coupling specificity requires that the polypeptide have the suitably rare and reactive residues in areas of the molecule that may be altered without loss of the molecule's biological activity. Alternatively, synthetic peptides with a modified residue can be synthesized such that specificity of linking is enhanced.

Cysteine residues may be replaced when they occur in parts of a polypeptide sequence where their participation in a cross-linking reaction would otherwise likely interfere with biological activity. When a cysteine residue is replaced, it is typically desirable to minimize resulting changes in polypeptide folding. Changes in polypeptide folding are minimized when the replacement is chemically and sterically similar to cysteine. For these reasons, serine is preferred as a re-placement for cysteine. When a cysteine residue is introduced, introduction at or near the amino or carboxy terminus is preferred. Conventional methods are available for such amino acid sequence modifications, whether the polypeptide of interest is produced by chemical synthesis or expression of recombinant DNA.

Coupling of the two constituents can be performed through a coupling or conjugating agent. Suitable intermo-lecular cross-linking reagents include for example, J-succinimidyl 3-(2-pyridyldithio) propionate (SPDP) or N,N'-(1,3-phenylene) bismaleimide (both of which are highly specific for sulfhydryl groups and form irreversible linkages); N, N'-ethylene-bis-(iodoacetamide) or other such reagent having 6 to 11 carbon methylene bridges (which relatively specific for sulfhydryl groups); and 1,5-difluoro-2, 4-dinitrobenzene (which forms irreversible linkages with amino and tyrosine groups). Other cross-linking reagents include: p,p'-difluoro-m,m'-dinitrodiphenylsulfon- e (which forms irreversible cross-linkages with amino and phenolic groups); dimethyl adipimidate (which is specific for amino groups); phenol-1,4-disul-fonylchloride (which reacts principally with amino groups); hexamethylenediisocyanate or diisothiocyanate, or azophenyl-p-diisocyanate (which reacts principally with amino groups); glutaraldehyde (which reacts with several different side chains) and disdiazobenzidine (which reacts primarily with tyrosine and histidine).

Cross-linking reagents may be homobifunctional, i.e., two functional groups that have the same reaction. A suitable homobifunctional cross-linking reagent is bismaleimidohexane ("BMH"). Cross-linking reagents may also be heterobifunctional. Heterobifunctional cross-linking agents have two different functional groups, for example an amine-reactive group and a thiol-reactive group, that will cross-link two proteins having free amines and thiols, respectively. Examples of heterobifunctional cross-linking agents are succinimidyl 4-(N-maleimidomethyl) cyclohexane-1-carboxylate ("SMCC"), m-maleimidobenzoyl-N-hydroxysuccinimide ester ("MBS"), and succinimide 4-(p-maleimidophenyl) butyrate ("SMPB"), an extended chain analog of MBS. The succinimidyl group of these cross-linkers reacts with a primary amine, and the thiol-reactive maleimide forms a covalent bond with the thiol of a cysteine residue. A hydrophilic moiety, such as a sulfonate group, may be added to the cross-linking reagent to improve its water solubility. Sulfo-MBS and sulfo-SMCC are examples of cross-linking reagents modified for water solubility.

Many cross-linking reagents yield a conjugate that may be non-cleavable under cellular conditions. However, some cross-linking reagents contain a covalent bond, such as a disulfide, that is cleavable under cellular conditions. For example, Traut's reagent, dithiobis (succinimidylpropionate) ("DSP"), and N-succinimidyl 3-(2-pyridyldithio) propionate ("SPDP") are well-known cleavable cross-linkers. The use of a cleavable cross-linking reagent permits the cargo moiety to separate from the transport polypeptide after delivery into the target cell. Direct disulfide linkage may also be useful.

Numerous cross-linking reagents, including the ones discussed above, are commercially available. Detailed instructions for their use are readily available from the commercial suppliers. A general reference on protein cross-linking and conjugate preparation is: Wong, Chemistry of protein conjugation and cross-linking, CRC Press (1991).

Chemical cross-linking may include the use of spacer arms. Spacer arms provide intramolecular flexibility or adjust intramolecular distances between conjugated moieties and thereby may help preserve biological activity. A spacer arm may be in the form of a polypeptide moiety that includes spacer amino acids, e.g. proline. Alternatively, a spacer arm may be part of the cross-linking reagent, such as in "long-chain SPDP" (Pierce Chem. Co., Rockford, IL, Cat. No. 21651 H).

Alternatively, the chimeric peptide can be produced as a fusion peptide that includes the cell-permeable se-quence and the caPCNA peptide variant sequence that can be expressed in known suitable host cells for large-scale production and purification. Fusion peptides, as described herein, can be formed and used in ways analogous to or readily adaptable from standard recombinant DNA techniques, as describe above.

Native peptides (in L-form) may be subject to degradation by natural proteases, the peptides disclosed herein may be prepared to include D-forms and/or "retro-inverso isomers" of the peptide. In this case, retro-inverso isomers of short fragments and variants of the peptide of caPCNA peptide variants disclosed herein are prepared. The caPCNA peptide variants can have one or more L-amino acids, D-amino acids, or a combination of both. For example, in various embodiments, the peptides are D retro-inverso peptides. The term "retro-inverso isomer" refers to an isomer of a linear peptide in which the direction of the sequence is reversed and the chirality of each amino acid residue is inverted. *See, e.g.,* Jameson et al., Nature, 368, 744-746 (1994); Brady et al., Nature, 368, 692-693 (1994). The overall result of combining D-enantiomers and reverse synthesis is that the positions of carbonyl and amino groups in each amide bond are exchanged, while the position of the side-chain groups at each alpha carbon is preserved. Unless specifically stated otherwise, it is presumed that any given L-amino acid sequence may be made into an D retro-inverso peptide by synthesizing a reverse of the sequence for the corresponding native L-amino acid sequence.

Suitable chemotherapy agents include for example, cyclophosphamide (CYTOXAN™), capecitabine (XELO-DA™), chlorambucil (LEUKERAN™), melphalan (ALKERAN™), methotrexate (RHEUMATREX™), cytarabine (CY-TOSAR-U™), fludarabine (FLUDARA™), 6-mercaptopurine (PURINETHOL™), 5-fluorouracil (ADRUCIL™), paclitaxel (TAXOL™), docetal, abraxane, doxorubicin (ADRIAMYCIN™), irinotecan (CAMPTOSAR™), cisplatin (PLATINOL™), carboplatin (PARAPLATIN™), oxaliplatin, tamoxifen (NOLVADEX™), bicalutamide (CASODEX™), anastrozole (ARIM-IDEX™), examestane, letrozole, imatinib (GLEEVEC™), rituximab (RITUXAN™), trastuzumab (HERCEPTIN™), gem-tuzumab, ozogamicin, interferon-alpha, tretinoin (RETIN-A™, AVITA™, RENOVA™), arsenic trioxide, bevicizumab (AVASTIN™), bortezombi (VELCADE™), cetuximab (ERBITUX™), erlotinib (TARCEVA™), gefitinib (IRESSA™), gemcitabine (GEMZAR™), lenalidomide (REVLIMID™), Serafinib, Sunitinib (SUTENT™), panitumumab (VECTIBIX™), pegaspargase (ONCASPAR™), and Tositumomab (BEXXAR™) and prodrugs or precursors or combinations thereof thereof.

Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C.

In an embodiment, the dosage of chemotherapy agents, when used in conjunction with the peptides of the disclosure, may be lower than the dosage used for a monotherapy. For example, doxorubicin when coadministered (either before or during or after) with caPCNA peptides of the disclosure, the dosage may be lowered by 25% or 35% or 50% or 60% or 75% or 80% of the standard dose. Depending on other factors, dosage may range from 300 mg/m² to 500 mg/m². Other suitable doses may be lower e.g., 20 mg/m², 50 mg/m², 100 mg/m², 150 mg/m², or 200 mg/m² or higher 400 mg/m², 450 mg/m², 500 mg/m², 550 mg/m², or 600 mg/m².

The peptides disclosed herein are also suitable for cancer patients undergoing radiotherapy (including all forms of ionizing radiations that are DNA damaging) and any other forms of cancer therapy. The amount of radiation used in radiation therapy is measured in gray (Gy), and may vary depending on the type and stage of cancer being treated. For curative cases, a typical dose for a solid epithelial tumor ranges from about 60 to 80 Gy, while lymphoma tumors are treated with about 20 to 40 Gy. Preventative (adjuvant) doses of radiation are typically around 45 - 60 Gy in 1.8 - 2 Gy fractions (e.g., for breast, head and neck cancers respectively.) Many other factors are considered by radiation oncologists when selecting a dose, including whether the patient is receiving chemotherapy or any other therapy, whether radiation therapy is being administered before or after surgery, and the degree of success of surgery. A typical fractionation schedule for adults is about 1.8 to 2 or to about 3 Gy per day. If the peptides as disclosed herein are used in combination with radiotherapy, then the radiation doses may reduced by, for example, 10%, 20%, 30%, 40%, 50%, 60%, and 75%. Modes of delivering radiotherapy include for example, conventional external beam radiotherapy (2DXRT), 3-dimensional conformal radiotherapy (3DCRT), stereotactic radiotherapy, image-guided radiation therapy (IGRT), and intensity-modulated radiation therapy (IMRT).

Particle therapy (Proton therapy) that uses energetic ionizing particles (protons or carbon ions) is also suitable. Radioisotope therapy (RIT) includes the use of radioisotopes to target tumor tissues. Examples include At²¹¹,I¹³¹,I¹²⁵, Y90 Re186 Re188 Sm153 Bi212 P32, Pb212 and radioactive isotopes of Lu.

Radioimmunotherapy includes the use of biologicals such as antibodies and a radioisotope. Ibritumomab tiux-etan (Zevalin™) is a monoclonal antibody anti-CD20 conjugated to a molecule of Yttrium-90. Tositumomab lodine-131 (Bexxar™) is a molecule of lodine-131 linked to the monoclonal antibody anti-CD20.

The peptide inhibitors disclosed herein are suitable augmenting agents that can be administered either prior to, during, and after administering a particular cancer therapy, e.g., chemotherapy or radiotherapy.

A "small molecule" refers herein to have a molecular weight below about 500 Daltons.

It is to be understood that cancers suitable for treatment using the peptides disclosed herein include, but are not limited to, malignancies such as various forms of glioblastoma, glioma, astrocytoma, meningioma, neuroblastoma, retinoblastoma, melanoma, colon carcinoma, lung carcinoma, adenocarcinoma, cervical carcinoma, ovarian carcinoma, bladder carcinoma, lymphoblastoma, leukemia, osteosarcoma, breast carcinoma, hepatoma, nephroma, adrenal carci-noma, or prostate carcinoma, esophageal carcinoma. If a malignant cell expresses csPCNA isoform, the compositions disclosed herein are capable of disrupting the interaction of caPCNA isoform with one or more proteins. Metastases of cancers are also treated by the peptide inhibitors disclosed herein. Any cell, whether cancerous or premalignant or precancerous, if it expresses cancer specific PCNA isoform, is suitable for reducing cellular proliferation or chemoprevention.

Non-peptidic compounds that mimic peptide sequences are known in the art (Meli et al. J. Med. Chem., 49:7721-7730 (2006), that describes methods of identifying nonpeptide small molecule mimics). Synthesis of non-peptide compounds that mimic peptide sequences is also known in the art (see, e.g., Eldred et al. J. Med. Chem., 37:3882, (1994); Ku et al. J. Med. Chem., 38:9, (1995); Meli et al. J. Med. Chem., 49:7721-7730 (2006)). Such nonpeptide compounds that mimic caPCNA-derived peptides or variants thereof disclosed herein that bind caPCNA are contemplated herein.

The term "peptidomimetic" or "peptide mimetic" refers to a chemical compound having small protein-like chain (peptide) that includes non-peptidic elements such as non-natural amino acids. Peptidomimetics are designed and synthesized with the purpose of binding to target proteins in order to induce or effect a particular change. Generally, a peptidomimetic functions by mimicking or antagonizing key interactions of the parent peptide structure that it was designed to mimic or antagonize. A peptidomimetic normally does not have classical peptide characteristics such as enzymatically cleavable peptidic bonds. For a general review of the various techniques available for design and synthesis peptide mimetics, see al-Obeidi et al., (1998), "Peptide and peptidomimetic libraries. Molecular diversity and drug design" Mol Biotechnol.; 9(3):205-23; and Houben-Weyl: Synthesis of Peptides and Peptidomemetics, Thieme Medical Publishers, 4th edition (2003).

As used herein, the terms "peptide mimetic," "peptidomimetic," and "peptide analog" are used interchangeably and refer to a synthetic chemical compound that has substantially the same structural and/or functional characteristics of caPCNA peptides or variants thereof disclosed herein. The mimetic can be either entirely composed of synthetic, non-natural analogues of amino acids, or, is a chimeric molecule of partly natural peptide amino acids and partly non-natural analogs of amino acids. The mimetic can also incorporate any amount of natural amino acid conservative sub-stitutions as long as such substitutions also do not substantially alter the mimetic's structure and/or inhibitory or binding activity. Routine experimentation will determine whether a mimetic is within the scope of the disclosure, i.e., that its structure and/or function is not substantially altered. Thus, a mimetic composition is within the scope if it is capable of specifically inhibiting caPCNA-mediated cellular proliferation.

Polypeptide mimetic compositions can contain any combination of nonnatural structural components, which are typically from three structural groups: a) residue linkage groups other than the natural amide bond ("peptide bond") linkages; b) non-natural residues in place of naturally occurring amino acid residues; or c) residues which induce sec-ondary structural mimicry, i.e., to induce or stabilize a secondary structure, e.g., a beta turn, gamma turn, beta sheet, alpha helix conformation, and the like.

A polypeptide can be characterized as a mimetic when all or some of its residues are joined by chemical means other than natural peptide bonds. Individual peptidomimetic residues can be joined by peptide bonds, other chemical bonds or coupling means, such as, e.g., glutaraldehyde, N-hydroxysuccinimide esters, bifunctional maleimides, N,N= dicyclohexylcarbodiimide (DCC) or N,N=diisopropylcarbodiimide (DIC). Linking groups that can be an alternative to the traditional amide bond ("peptide bond") linkages include, e.g., ketomethylene (e.g.,-C=O-CH₂ for -C=O-NH--), ami-nomethylene (CH₂--NH), ethylene, olefin (CH=CH), ether (-CH₂-O), thioether (CH₂-S), tetrazole (CN₄), thiazole, retro-amide, thioamide, or ester (see, e.g., Spatola (1983) in Chemistry and Biochemistry of Amino Acids, Peptides and Proteins, Vol. 7, pp 267-357, A Peptide Backbone Modifications, Marcell Dekker, NY).

A polypeptide can also be characterized as a mimetic by containing all or some non-natural residues in place of naturally occurring amino acid residues. Nonnatural residues are well described in the scientific and patent literature; a few exemplary nonnatural compositions useful as mimetics of natural amino acid residues.

In another embodiment, peptides capable of disrupting caPCNA interaction include peptides of amino acid sequences with one or more amino acid substitutions that include about +3 contiguous or non contiguous additional amino acids on the NH₂ terminus of LGIPEQEY and about +9 contiguous or non contiguous amino acids on the COOH terminus of LGIPEQEY. For example, some of these peptides include amino acid sequences of QLGIPEQEYSC (+1-NH2 terminus, +2 -COOH terminus), VEQLGIPEQEY (+3-NH2 terminus), LGIPEQEYSCVVK (+5-COOH terminus), LGIPEQEYSCVVKMPSG (+9-COOH terminus), EQLGIPEQEY (+2-NH2 terminus), QLGIPEQEY (+1-NH2 terminus), LGIPEQEYSCVVKMPS (+8-COOH terminus), LGIPEQEYSCVVKMP (+7-COOH terminus), LGIPEQEYSCVVKM (+6-COOH terminus), LGIPEQEYSCVV (+4-COOH terminus), LGIPEQEYSCV (+3-COOH terminus), LGIPEQEYSC (+2-COOH terminus), LGIPEQEYS (+1-COOH terminus) and combinations of the additional NH₂ and COOH termini amino acids that flank LGIPEQEY. Amino acid mutations including substitutions that do not affect the specificity of the peptides to generate csPCNA specific antibodies are within the scope of this disclosure. One or more of the amino acid residues in the peptides may be replaced with an amino acid analog or an unnatural or non-standard amino acid.

Dosage of the caPCNA-derived peptide variants depend on the efficacy of the peptides, stability of the peptides *in vivo,* mode of administration, the nature of cancer being treated, body weight, age of the patient and other factors that are commonly considered by a skilled artisan. For example, dosage of caPCNA-derived peptide variants drug can range from about 0.1-10.0 microgram (mcg)/kg body weight or from about 0.2-1.0 mcg/kg body weight or from about 0.5-5.0 mcg/kg body weight or from about 10.0-50.0 mcg/kg body weight. Depending on the toxicity effects and tumor killing capability, the dosage can also range from about 1.0-10.0 mg/kg body weight and from about 0.1-1.0 mg/kg body weight. The amount of the inhibitor that is administered to the subject can and will vary depending upon the type of inhibitor, the subject, and the particular mode of administration. Those skilled in the art will appreciate that dosages may also be determined with guidance from Goodman & Goldman's The Pharmacological Basis of Therapeutics, Ninth Edition (1996), Appendix II, pp. 1707-1711 and from Goodman & Goldman's The Pharmacological Basis of Therapeutics, Tenth Edition (2001), Appendix II, pp. 475-493.

Administration of the compositions disclosed herein may be via any route known to be effective by the physician of ordinary skill. Parenteral routes include intravenous, intramuscular, subcutaneous, and intraperitoneal routes of ad-ministration. Intravenous, intramuscular, and subcutaneous routes of administration of the compositions disclosed herein are suitable. For parenteral administration, the peptides disclosed herein can be combined with phosphate buffered saline (PBS) or any suitable pyrogen-free pharmaceutical grade buffer that meets FDA standard for human subject administration. As used herein, "pharmaceutically acceptable carrier" includes any and all solvents, diluents, or other liquid vehicle, dispersion or suspension aids, surface active agents, isotonic agents, thickening or emulsifying agents, preservatives and the like, as suited to the particular dosage form desired. Remington's Pharmaceutical Sciences, 20th Edition, A.R. Gennaro (Williams and Wilkins, Baltimore, MD, 2000) discloses various carriers used in formulating pharmaceutical compositions and known techniques for the preparation thereof. Solutions or suspensions of the compositions described herein can also include a sterile diluent, such as water for injection, saline solution, fixed oils, polyethylene glycols, glycerine, propyleneglycol or other synthetic solvents; chelating agents, such as EDTA; buffers, such as acetates, citrates or phosphates; and agents for the adjustment of tonicity, such as sodium chloride or dextrose. A parenteral preparation of the compositions can be enclosed in ampoules, disposable syringes or multiple dose vials made of glass or plastic, in accordance with standard practice in the field. The compositions disclosed herein can be stored as a lyophilized sterile powder in vials containing for reconstitution and the unreconstituted product may be stored at -20°C.

Agents administered parenterally, i.e., intravenously, intramuscularly, etc., may include a sterile diluent such as water, saline solution, a pharmaceutically acceptable polyol such as glycerol, propylene glycol, polyethylene glycols, or other synthetic solvents; an antibacterial and/or antifungal agent such as benzyl alcohol, methyl paraben, chlorobutanol, phenol, thimerosal, and the like; an antioxidant such as ascorbic acid or sodium bisulfite; a chelating agent such as etheylenediaminetetraacetic acid; a buffer such as acetate, citrate, or phosphate; and/or an agent for the adjustment of tonicity such as sodium chloride, dextrose, or a polyalcohol such as mannitol or sorbitol. The pH of the solution may be adjusted with acids or bases such as hydrochloric acid or sodium hydroxide. Preparations for oral administration generally include an inert diluent or an edible carrier. They may be include a pharmaceutically compatible binding agent such as microcrystalline cellulose, gum tragacanth or gelatin; an excipient such as starch or lactose; a disintegrating agent such as alginic acid, Primogel, or corn starch; a lubricant such as magnesium stearate or sterotes; a glidant such as colloidal silicon dioxide; a sweetening agent such as sucrose or saccharin; and/or a flavoring agent such as peppermint, methyl salicylate, or citrus flavoring. Oral preparations may be enclosed in gelatin capsules, compressed into tablets, or prepared as a fluid carrier. For administration by inhalation, the agent is generally delivered in the form of an aerosol spray from a pressurized container or dispenser that contains a suitable propellant, e.g., a gas such as carbon dioxide, or a nebulizer. For topical (e.g., transdermal or transmucosal) administration, penetrants appropriate to the barrier to be permeated are generally included in the preparation. Transmucosal administration may be accomplished through the use of nasal sprays or suppositories, and transdermal administration may be via ointments, salves, gels, patches, or creams as generally known in the art.

Peptides and other compositions disclosed herein can be administered via any suitable means. For example, the peptide compositions may be diluted in saline or any suitable buffer and administered directly intravenously. For example, the peptide compositions can be encapsulated in liposomes and administered intravenously of by any suitable method. For example, the peptide compositions can be delivered by an extended release drug delivery system known to one of ordinary skill in the art. Other modes of targeting tumors are also suitable. For example, U.S. patent application publication US20050008572 (Prokop et al.,) discloses methods and compositions relating to nanoparticular tumor tar-geting and therapy. U.S. patent application publication US20030212031 (Huang et al.,) discloses stable lipid-comprising drug delivery complexes and methods for their production.

Replication defective viral expression vectors (e.g., lentivirus; adenovirus, adeno-associated virus, herpes virus, and others) capable of expressing the peptides disclosed herein are also suitable delivery systems. Other nucleic acid delivery systems such as retroviral vectors, adenovirus vectors, adeno-associated virus vectors, alphavirus vectors, Semliki Forest virus-based vectors, Sindbis virus-based vectors are also useful, See, Schlesinger and Dubensky (1999) Curr. Opin. Biotechnol. 5:434-439 and Ying et al. (1999) Nat. Med. 5(7):823-827.

caPCNA-derived peptides disclosed herein are expressed from a suitable viral vector. cDNA sequence of PCNA gene is found for example, in Travali et al., (1989), J. Biol. Chem. 264 (13), 7466-7472 and also in several GenBank entries such as for example, NM_002592 and NM_182649. Standard cloning methods based on PCR and site-directed mutagenesis techniques are used to engineer one or more Ala substations or other conserved mutations in the coding region of caPCNA regions for expressing the variant peptides in a cancer cell.

In addition, the term "pharmaceutically effective amount" or "therapeutically effective amount" refers to an amount (dose) effective in treating a patient, having, for example, breast cancer. For example, in vitro test conditions, a suitable dose would kill at least 50% of cancer cells. It is also to be understood herein that a "pharmaceutically effective amount" may be interpreted as an amount giving a desired therapeutic effect, either taken in one dose or in any dosage or route, taken alone or in combination with other therapeutic agents. In the case of the present disclosure, a "pharma-ceutically effective amount" may be understood as an amount of caPCNA-derived peptide variants which may for example, suppress (e.g., totally or partially) the inhibition of caPCNA and one or more of its interacting partners or reduce tumor growth or reduce cancer cell proliferation.

The following examples are exemplary and are not intended to limit the scope of the disclosure.

### EXAMPLES

### EXAMPLE 1

### Cytotoxic effects of cell-permeable caPCNA-derived peptide variants on breast cancer cells

caPCNA derived peptides (Table 1 and Table 2) introduced into breast cancer cells compete with PCNA for binding of its partner protein and, thereby, prohibit full-length PCNA from interacting with them. This peptide represents part of the inter-domain connecting loop (IDCL) of PCNA and competes with PCNA for binding to proteins such as polymerase δ, xeroderma pigmentosum group G (XPG), or FEN-1 which are necessary to carry out proper DNA replication and repair. A decrease in a cell's ability to properly replicate and repair its DNA would eventually lead to apoptotic cell death. This is likely to lead to dysfunctional DNA replication and repair, resulting in the death of cancer cells.

A nonaarginine-linked caPCNA derived peptide (R9-caPep) was used to test the effect of disrupting caPCNA interaction. One group of cell-penetrating peptides relies on positively charged amino acids for cellular uptake. Polyarginines have been shown to be more efficient than polylysines and polyhistidines at promoting cellular uptake. Specifically, D-arginine sequences are more efficient than L-arginine sequences. Thus, a D-nonaarginine linked caPeptide was created with the sequence RRRRRRRRRCCLGIPEQEY (R9-caPep).

U937 monocytic lymphoma cells grow in suspension and have been shown to be a model cell line to use in therapeutic peptide evaluation since the cell line grows very quickly and provides highly reproducible results. Western blot analysis indicated that this cell line contains both the acidic (caPCNA) and basic (nmPCNA) isoforms of PCNA. First, it was tested whether or not the R9 sequence alone had any cytotoxic effect in U937 cells. The U937 cells were treated with up to 100mM of the R9 sequence for 24 hours. The R9 sequence did not promote apoptosis in these cells (FIG. 2A). Cells were then treated with increasing concentrations of unlinked caPeptide to ensure that it did not possess cytotoxic activity without the N-terminal arginine sequence. Up to 200mM of caPeptide was added to the U937 cells for 24 hours. caPeptide alone did not have any cytotoxic activity (FIG. 2A).

To test cellular uptake and nuclear localization of R9-caPep, an N-terminal fluorescein isothiocyanate (FITC) residue was attached to allow microscopic visualization of the peptide's cellular location. The U937 cells were treated with 10mM of FITC-R9-caPep for 24 hours. The cells were then collected onto a poly-L-lysine coated slide by cytospin and mounted with a media containing the DNA staining dye 4'-6-Diamidino-2-phenylindole (DAPI), which complexes with double stranded DNA and indicates the location of the nucleus. A Leica DM500 fluorescent microscope was used to visualize the staining. FIG. 2B 4 shows the DAPI stained nuclei (blue) and the FITC labeled R9-caPep (green) do overlap indicating that the R9-caPep was internalized by the cells and was accessible to the nucleus.

Once it was established that the R9 sequence promoted cellular uptake of the peptide, increasing amounts of R9-caPep were added to U937 cells to determine if the peptide's uptake would have any cytotoxic effect. R9-caPep concentrations above 20mM were observed to induce a marked increase in apoptotic cells with almost 93% of cells being apoptotic in the presence of 50uM of R9-caPep (FIG. 2A).

See FIG. 3 that shows nuclear localization of R9-caPep. U937 cells were treated with FITC-R9-caPep and collected on coated slides by cytospin. Fluorescent images for the DAPI stained nuclei (left) and FITC-R9-caPep (right) were overlaid (center) showing cellular uptake and nuclear localization of R9-caPep. Boxes indicate intact cells.

To verify that the cytotoxicity of the R9-caPep was due to the PCNA peptide sequence itself, a scrambled peptide using the same eight amino acids, R9-Scrambled, was prepared (Table 3). Treatment of U937 cells with R9-Scrambled resulted in little cytotoxicity (Table 3). These data indicated that the cytotoxicity of the R9-caPep was due to the caPCNA peptide sequence and was not due to any potential cellular damage from uptake of peptide.

caPeptide was incubated with U937 cells for 24 hours but no increase in apoptosis was detected. Since most peptides are not passively transferred into cells, a nonaarginine linker was attached to the N-terminal portion of caPeptide to create R9-caPep. Fluorescent visualization of the R9-caPep revealed that cellular uptake occurs and that the peptide can locate to the nucleus. The R9 sequence alone was tested for cell toxicity and it did not cause an increase apoptosis. However, R9-caPep caused a marked increase in apoptosis, especially at concentrations above 20mM with almost 100% cell death at 50mM. When the amino acid sequence of caPeptide was scrambled and attached to the R9-linker to create R9-Scrambled, no cytotoxicity was detected.

Further as shown in Table 6, the caPCNA inhibiting peptide was substantially specific in killing cancer cells as compared to non-malignant cells. In order to demonstrate that the work with U937 cells was directly relatable to our breast cancer studies, the relative sensitivity of a breast cancer cell line (MCF7) and a non-malignant breast cell line (MCF-10A) to the R9-caPeptide was determined. The R9-caPeptide was evaluated for cytotoxic effects in non-malignant (MCF 10A) and malignant (MCF 7) breast cells (Table 6). The cells were treated with 50 mM R9-caPeptide for 48 hours and the extent of cell death was evaluated by flow cytometry. R9-caPeptide was observed to be approximately five times more cytotoxic for malignant breast cells (MCF7) than non-malignant breast cells (MCF10A). The significant level of cytotoxicity in breast cancer cells is likely due to enhanced binding interaction of caPeptide with specific DNA repair proteins in these cells, as indicated for example by the observed preferential binding of caPCNA for XPG protein, leading to an inhibition of DNA repair in these cells, and ultimately cell death.

Therefore, this example demonstrated that R9-caPep selectively disrupted the caPCNA interaction with one or more of its binding partners and resulted in cell death.

### EXAMPLE 2

### R9-caPep Augments the Action of Doxorubicin and Sensitizes Tumor Cells for Chemotherapy

Since R9-caPep was effective at killing cancer cells, it was tested whether combining the peptide with a DNA damaging chemotherapeutic agent would allow for a lower concentration of the drug to be used. Doxorubicin is a DNA damaging agent used to treat many types of solid tumors and acute leukemias. It is a very effective drug but one of its main side effects is cardiomyopathy. Due to the damage that doxorubicin causes to the heart, patients are only allowed to receive a total lifetime dose of less than 500 mg/sq m. If lower doses of doxorubicin, combined with a cytotoxic dose of R9-caPep, can cause the same amount of cell death as high concentrations of doxorubicin alone, it would be possible to use less doxorubicin which would likely reduce harmful side effects. U937 cells were treated with 25mM of R9-caPep together with increasing concentrations of doxorubicin for 24 hours. The combination of R9-caPep and doxorubicin reduces the median inhibitory concentration 50 (IC₅₀) of doxorubicin from approximately 140nM to 110nM (FIG. 2B). The effect that R9-caPep has in combination with doxorubicin seems to be more pronounced at lower concentrations. When the U937 cells are treated with 100nM of doxorubicin 30% cell death is achieved; however, only 25 nM of doxorubicin is needed to achieve the same level of cytotoxicity when combined with 25mM of R9-caPep. Although there is not a large reduction in the IC₅₀ when 25mM of R9-caPep is administered in combination with doxorubicin, any reduction in the amount of doxorubicin needed could potentially reduce the harmful side effects the drug would have on a patient.

Cell types (e.g., breast, colon, lung, leukemias, and the like) expressing the caPCNA isoform are sensitive to the caPCNA-derived peptides, as these peptides' core structure resemble the a unique structure within the caPCNA isoform (and absent from the non-malignant (nm) PCNA isoform due to the lack of post-translational modification of one or more of the acidic amino acids in a stretch of 8 amino acids (126-133) in the caPCNA isoform. The sensitivity of Leukemia cells and neuroblastoma cells to these peptides were tested. Colon cancer cells, cervical cancer cells, ovarian cancer cells, prostate cancer cells, lung cancer cells, esophageal cancer cells, liver cancer cells, pancreatic cancer cells, breast cancer cells, melanoma cancer cells, (and other forms of cancer) all express the caPCNA isoform. Thus, all of these cancer types and any other cancer type that expresses caPCNA are sensitive to the cytotoxic effects of the caPCNA-derived peptides or variants thereof on these cancer cells.

Thus, R9-caPep (or any other caPCNA-based peptides or variants thereof disclosed herein) is useful as a therapeutic agent either alone or in combination with other DNA damaging agents. The harmful side effects that result from treatment with chemotherapeutic agents like doxorubicin is substantially reduced if the drug is used in combination with R9-caPep. This example demonstrates that R9-caPep is capable of augmenting the cytotoxic effects of a DNA damaging agent and also likely renders resistant tumors sensitive to cancer therapy agents and radiation therapy.

### EXAMPLE 3

### Identification of Interacting Amino Acids for the Cytotoxic Action of R9-caPep

The cancer associated epitope of PCNA is also part of the flexible IDCL region of PCNA. This region of PCNA is considered to have a disordered structure. Disordered regions in proteins are usually associated with protein-to-protein interaction sites. The cytotoxic effect of R9-caPep indicates that it interacts with full length PCNA's binding partners and inhibiting the normal processes that occur via PCNA binding. To determine which amino acids in the R9-caPep sequence are necessary for binding of the peptide to these proteins, peptide constructs were generated that mutated each aminoacid individually to an alanine (Table 3). Each alanine mutant peptide of caPCNA was used to treat U937 cells for 24 hours. An amino acid that is important to protein binding would presumably show decreased cell cytotoxicity when it is substituted with an alanine. Those amino acids which are not critical for binding could be anticipated to show a cytotoxic level similar to that of the unsubstituted peptide. For a comparison of the potential cytotoxic effect of each of the alanine substituted R9-linked caPeptides, U937 cells were treated with 30mM of peptide. This was the concentration at which R9-caPep resulted in almost 50% cell death (FIG. 2A). The results of this study are shown in Table 3. It was observed that peptides containing an alanine substituted at position aa126 or aa133 show little cytotoxic activity and therefore were likely important for the peptide's activity. The peptide with an alanine substituted at amino acid position 128 exhibited a decrease in cytotoxicity though not to the extent observed with substitutions at positions 126 or 133. Surprisingly, alanine substitutions at amino acid positions 129, 131, or 132 were observed to significantly increase the cytotoxic activity of the peptide.

Thus, this example demonstrates that amino acid substitutions to caPCNA derived peptide provide various peptide variants of therapeutic use.

### EXAMPLE 4

### Molecular Modeling of the Observed caPeptide Alanine Substituted Effects on Cvtotoxicitv

To gain insight into the potential relationships between the observed cytotoxic effects of the alanine substituted caPeptides and their ability to affect the recognition of the alternated peptide by PCNA partners, *in silico* molecular modeling studies were performed. To perform these studies, the interaction of PCNA and its known binding partner FEN-1 were modeled. FEN-1 is one of the few PCNA binding partners for which the x-ray crystal structure is actually known and, therefore, would facilitate the modeling study. The crystal structure of the PCNA homotrimer bound to three FEN-1 molecules was developed.

Each FEN-1 molecule binds to its corresponding PCNA monomer in a different orientation with PCNA molecules A, B, and C binding to FEN-1 molecules X, Y, and Z respectively. The difference in the orientation of each FEN-1 monomer is clearly shown when corresponding PCNA/FEN-1 monomer pairs A-X and C-Z are overlaid. However, the C-terminal tail regions of both the X and Z chains of FEN-1 (aa330-380) interact with IDCL portion of PCNA. (see FIG. 5). When this complex was rotated 90° and the PCNA molecule was depicted in a space-filling model, it became apparent that the aa126-133 peptide formed a cavity that fits around FEN-1 amino acid residues that interacted in the area of the cavity (FIG. 6). This cavity was flanked by aa126 and aa133 of PCNA, the amino acids which were shown to be important for the cytotoxic activity of R9-caPep by the alanine mutation analysis (Table 3). A closer look at the amino acid R groups of aa126-133, in relation to the cavity, reveals that aa126, aa128, and aa133 face the cavity and provide contact points for the PCNA/FEN-1 interaction (FIG. 7). However, the R groups of aa127 and aa130 point away from the pocket and do not appear to be involved in PCNA/FEN-1 binding (FIG. 7). Of the amino acids that were shown to enhance cytotoxic activity by the alanine mutation analysis, aa129 and aa131 face the cavity and aa132 points away from the cavity. When these amino acids are mutated to an alanine, a conformational change occurs which may cause the cavity to close more tightly around the binding partner thereby creating a stronger interaction.

Although FEN-1 had been used as the model for this study, the cavity formed by aa126-133 is also important for binding of PCNA to many of its partners. FIG. 7 lists several PCNA binding partners and the conserved sequences used to interact with PCNA. When the five amino acid consensus binding sequence of each peptide or protein was fit into the PCNA space-filling model where it is reported to interact, they all overlay in the pocket region formed by caPeptide. Although there are many other amino acid interacting points between PCNA and its binding partners, the cavity formed by the caPeptide region may be necessary for proper binding and the initiation of the cellular processes produced by the interaction.

*In silico* molecular modeling was performed to further investigate the contribution of the PCNA amino acid sequence to R9-caPep cytotoxicity. The positions of aa126-133 were determined from the crystal structure of PCNA bound to FEN-1. The analysis revealed that aa126-133 of PCNA form a cavity in which a conserved five amino acid sequence of several of PCNA's binding partners fit. The amino acids determined to be important for the activity of R9-caPep from the alanine mutation analysis were the same amino acids which face the cavity and possibly provide contact points between PCNA and the binding partner (aa126, aa128, and aa133). Those amino acids that are not important for the cytotoxic activity of R9-caPep face away from the pocket and do no appear to participate in the binding of PCNA to its partner (aa127 and aa130). Of the amino acids that cause an increase in cytotoxicity, aa129 and aa131 face the pocket and aa132 is turned away from the pocket.

The cellular cytotoxicity shown by R9-caPep is thought to be caused by the peptide acting as decoy for PCNA's binding partners. Since the peptide mimics the IDCL of full length PCNA, it could bind to proteins such as XPG, polymerase δ, and FEN-1 thus preventing them from binding to intact PCNA. Sequestration of these proteins by the peptide would prevent proper DNA replication and repair and lead to cell death. Mutation of certain amino acids (aa126, aa128, and aa133) of R9-caPep decreases its ability to be recognized by PCNA's binding partners and reduces the peptides ability to disrupt PCNA-partner interactions thus leading to decreased cell death. The mutation of amino acids 129, 131, and 132 to an alanine may cause a conformational change which allows the peptide to bind more tightly to potential partners thus causing increased cytotoxicity.

### EXAMPLE 5

### Chemoprevention using caPCNA peptides and variants thereof

caPCNA peptides and variants thereof are used as chemo-preventative agents for chemoprevention, in which the peptides are effective in limiting the transformation of, for example, pre-cancerous cells into cancerous cells or detectable tumors. Due to the ability of the caPCNA derived peptides or the variants thereof to selectively kill cancer cells while having a minimal effect on the viability of non-cancer cells, these peptides or the variants are useful in preventing carcinogenesis. This approach is based on the observation that because the expression of the cancer specific isoform of PCNA (caPCNA) is a relatively early event in the transformation process of cells leading to a malignant phenotype, blocking the interaction of caPCNA with one or more of its binding partners at an early stage, results in an effective chemoprevention.

Any tissue that is capable of expressing caPCNA isoform at an early stage prior to the development of a detectable tumor mass or a malignant phenotype, is suitable for chemopreventive therapy using the caPCNA peptides and the variants thereof disclosed herein. Depending on the toxicity of the caPCNA derived peptides and the variants disclosed herein, a skilled artisan would readily recognize and optimize the dosage ranges to implement an effective chemoprevention treatment plan. For example, if the caPCNA-derived peptides and the variants disclosed herein are not significantly toxic to normal cells, then higher doses generally used for diagnosed cancer patients are used for chemoprevention in high-risk individuals. If the caPCNA-derived peptides and the variants disclosed herein are somewhat toxic, then depending on the toxicity, a relatively lower dosage as compared to a cancer therapy dose is used for the chemopreventative treatment. In addition, the dosage may vary for example, depending on the cancer type, the stage at which the individual is being treated, mode of delivery, and any other clinical data such as age, general health of the individual and the like.

For example, caPCNA expression is readily observed in Chronic Myelogenous Leukemia (CML) patients, staged as being 3 years from undergoing conversion to the acute phase of the disease. Thus, the caPCNA peptides and the variants are also capable of reducing the viability of these types of early transformed cells, and prior to development of a clinically recognized or detectable cancer.

Generally, the caPCNA-derived peptides or the variants are administered by a suitable method to eliminate or reduce the number of early transformed cells that are likely to express the caPCNA isoform and likely to become cancerous. This type of preventive administration is useful in the clinical management of individuals belonging to "high risk" groups such as those with familial types of cancer including those of epithelial (e.g., various adenocarcinomas) or mesothelial origin (e.g., various sarcomas), and/or individuals exposed to various environmental toxicants derived from either their work or home or regional environments, and/or individuals treated for a cancer and who are being monitored for recurrence of the disease (i.e., breaking out of remission). Because only cancer cells or cells undergoing the trans-formation process express the caPCNA isoform, these transformed cells that are sensitive to the caPCNA-derived peptides and variants are selectively targeted and killed, while the viability of non-transformed cells is not likely to be substantially affected by the caPCNA derive-peptides and variants thereof.

Long term benchmarks for clinical benefits of using the caPCNA-derived peptides and variants as chemo-preventative agents would include for example, decrease in the number of "high risk" patients developing particular malignancies, reductions in the number of cancer recurrences following excision of primary cancer lesions, reductions in the number of occurrences of tumors resulting from exposure to carcinogenic or mutagenic substances encountered within the home, work, or regional environment for individuals and reductions in the number of occurrences of secondary tumors resulting from chemotherapeutic administration of agents to treat unrelated cancers.

For example, co-expression of both p16^{INK4} (Bean et al., (2007), Clin. Cancer Res.; 13(22), 6834-41) and caPCNA in what appears to be non-cancer cells serves a marker for identification pre-cancerous or pre-malignant cells for chemoprevention. For example, the expression of the protein p^{16INK4} indicates that expression of this protein precedes transformation of the cell expressing p16^{INK4} to a cancer phenotype. Individual molecular changes occurring early in the transformation process may predispose these cells to becoming cancer cells, and that identification of such a change in gene expression is an indicator (marker) of the pending morphologically recognizable transformation event at an early stage. Thus, early molecular alterations associated with the transformation event, while having no discernable effect on the morphology of the cells beginning the transformation process, nonetheless are geared towards undergoing trans-formation into a cancer cell. Since expression of caPCNA is universally associated with the malignant transformation process (all cancer cells examined to date express the caPCNA isoform), and occasionally one or more normal breast epithelial cells within a duct that is adjacent (within 1 cm) to breast cancer epithelial cells will also express caPCNA. Co-expression of p16^{INK4} in these caPCNA expressing cells, and absence from the non-expressing cells, may serve as a marker for the transformation process. Such "normal" cells expressing caPCNA are suitable targets to use the compositions disclosed herein to selectively kill the caPCNA expressing "normal" cells before they are malignant.

Therefore, the caPCNA-derived peptides or the variants disclosed herein present a viable chemopreventative option to reduce the overall occurrence of cancer in tissue types that are likely to express caPCNA isoform at an early stage. A practitioner or a clinician can readily determine if the individual or a tissue biopsy expresses caPCNA by using a caPCNA-specific detection method e.g., caPCNA-specific antibodies disclosed in international patent application pub-lication WO2006/116631 or based on caPCNA isoform post-translational modifications disclosed in international patent application publication WO 2007/002574.

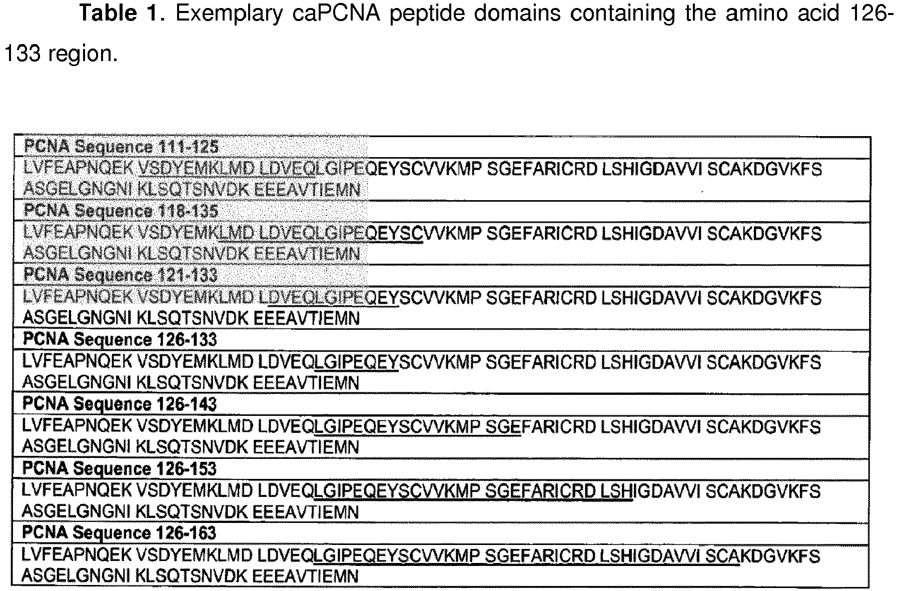

The regions containing the 126-133 sequence are shown as underlined.

**Table 2. Exemplary caPCNA peptide domains containing the amino acid 126-133 region.**

| Name | Sequence |
|---|---|
| R9 Alone | RRRRRRRRR |
| caPeptide | LGIPEQEY |
| R9-caPep | RRRRRRRRRCCLGIPEQEY |
| R9-L126A | RRRRRRRRRCCAGIPEQEY |
| R9-L127A | RRRRRRRRRCCLAIPEQEY |
| R9-L128A | RRRRRRRRRCCLGAPEQEY |
| R9-L129A | RRRRRRRRRCCLGIAEQEY |
| R9-L130A | RRRRRRRRRCCLGIPAQEY |
| R9-L131A | RRRRRRRRRCCLGIPEAEY |
| R9-L132A | RRRRRRRRRCCLGIPEQAY |
| R9-L133A | RRRRRRRRRCCLGIPEQEA |
| FITC-R9-caPep | FITC-RRRRRRRRRCCLGIPEQEY |

**Table 3. Cytotoxic effects of R9-linked caPCNA peptides.**

| **Peptide designation** | **Arginine-linked caPCNA peptide and the respective alanine substitutions** | | | | | | | | | | **% cancer cell death** |
|---|---|---|---|---|---|---|---|---|---|---|---|
| 126-133 peptide | 126- | **-L** | **G** | **I** | **P** | **E** | **Q** | **E** | **Y** | **-133** | |
| Unsub. peptide | R9- | 1 | 2 | 3 | 4 | 5 | 6 | 7 | 8 | | 43±10 |
| A1-sub. peptide | R9- | A | 2 | 3 | 4 | 5 | 6 | 7 | 8 | | 8±9 |
| A2-sub. peptide | R9- | 1 | A | 3 | 4 | 5 | 6 | 7 | 8 | | 47±13 |
| A3-sub. peptide | R9- | 1 | 2 | A | 4 | 5 | 6 | 7 | 8 | | 21±12 |
| A4-sub. peptide | R9- | 1 | 2 | 3 | A | 5 | 6 | 7 | 8 | | 80±7 |
| A5-sub. peptide | R9- | 1 | 2 | 3 | 4 | A | 6 | 7 | 8 | | 45±14 |
| A6-sub. peptide | R9- | 1 | 2 | 3 | 4 | 5 | A | 7 | 8 | | 93±7 |
| A7-sub. peptide | R9- | 1 | 2 | 3 | 4 | 5 | 6 | A | 8 | | 88±4 |
| A8-sub. peptide | R9- | 1 | 2 | 3 | 4 | 5 | 6 | 7 | A | | 7±6 |
| Negative control | Scrambled peptide | | | | | | | | | | 5±4 |

| | | | | | | | | | | | |
|---|---|---|---|---|---|---|---|---|---|---|---|
| R9 refers to a cell penetrating peptide that includes a polyarginine sequence e.g., nine contiguous arginine residues. Alanine substitutions in the caPCNA peptide affect its cytotoxic action. U937 cells were treated with each alanine substituted peptide and the scrambled peptide and % cell death evaluated by flow cytometry. The scrambled peptide and alanine substitutions at positions aa126 or aa133 show reduced cytotoxic activity. Substitutions at aa129, aa131, or aa132 cause an increase in cytotoxicity of the peptide. Substitutions that result in a neutral change include aa127 and aa130. Substitution at aa128 results in a decrease in cytotoxic action but not to the levels of aa126 or aa133. | | | | | | | | | | | |

**Table 4: Cell permeable or cell-penetrating peptides**

| **Name** | **Peptide sequence** | **References** (each is incorporated by reference in its entirety) |
|---|---|---|
| R9 | RRRRRRRRR | |
| Penetratin™ | RQIKIWFQNRRMKWKK | U.S. Pat. No. 5,888,762 |
| Tat | GRKKRRQRRRPPQ | U.S. Pat. Nos. 5,804,604 and 5,674,980 |
| TAT (47 - 57) | YGRKKRRQRRR | Wender, PA. et al. Proc. Natl. Acad. Sci. USA 97, 13003 (2000) |
| Tat (48 - 57) | GRKKRRQRRR | Hottiger, M. and G. Nabel, J. Virol. 72, 8252 (1998). |
| Tat (Npys) | YGRKKRRQRRRGGG-C(Npys)-NH2 | |
| Transportan™ | GWTLNSAGYLLGKINLKALAALAKKIL | |
| VP22 | DAATATRGRSAASRPTERPRAPARSASRPRRPVD | WO 97/05265 |
| MAP | KLALKLALKALKAALKLA | |
| KALA | WEAKLAKALAKALAKHLAKALAKALKACEA | |
| ppTG20 | GLFRALLRLLRSLWRLLLRA | |
| Trimer | VRLPPP | |
| P1 | MGLGLHLLVLAAALQGAWSQPKKKRKV | |
| MPG | GALFLGFLGAAGSTMGAWSQPKKKRKV | |
| Pep-1 | KETWWETWWTEWSQPKKKRKV | Fischer, R. et al. Chem. Bio. Chem. 6, 2126 (2005). |
| hCT | LGTYTQDFNKFHTFPQTAIGVGAP | |
| C105Y | CSIPPEVKFNKPFVYLI | Rhee and Davis, (2006) J. Biol. Chem. 281, 1233 |
| 105Y | SIPPEVKFNKPFVYLI | Boland, K. et al. J. Biol. Chem. 270,28022 (1995) |
| Lipid Membrane Translocating Peptide | KKAAAVLLPVLLAAP and its D-isomer | |
| Nuclear localization | PKKKRKV | |
| RVG | YTIWMPENPRPGTPCDIFTNSRGKRASNGGGG | Kumar, P. et al. Nature 448, 39 (2007). |
| Transdermal Peptide | ACSSSPSKHCG | Chen, Y. et al. Nat. Biotechnol. 24, 455 (2006). |
| Antennapedia Leader Peptide (CT) | KKWKMRRNQFWVKVQRG | Kanovsky, M. et al. Proc. Natl. Acad. Sci. 98, 12438 (2001). |
| Antennapedia Peptide | RQIKIWFQNRRMKWKK | Jain, M. etal. CancerRes. 65, 7840 (2005). |
| SynB1 | RGGRLSYSRRRFSTSTGRA | |

The peptides listed above can be synthesized or are commercially available (e.g., AnaSpec, San Jose, CA) with and NH₂ moiety for coupling with the peptide variants disclosed herein.

**Table 5: List of exemplary amino acid conserved substitutions**

| **Amino Acid** | **Code** | **Conserved Substitutions** |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, β-ala, Acp |
| Isoleucine | I | D-Ille, Val, D-Val, Leu, D-Leu, Met, D-Met |
| Leucine | L | D-Leu, Val, D-Val, Met, D-Met |
| Lysine | K | D-Lys, Arg, D-Arg, homo-Arg, D-homo-Arg, Met, D-Met, Ile, D-Ile, Orn, D-Om |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4 or 5-phenylproline |
| Proline | P | D-Pro, L-I-thioazolidine-4-carboxylic acid, D- or L-1-oxazolidine-4-carboxylic acid |
| Serine | S | De-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Lie, D-Lle, Met, D-Met |

**Table 6: Preferential killing of cancer cells by R9-caPeptide**

| **Cell line** | **% Dead Cells** |
|---|---|
| MCF 10A (non-malignant) | 18.2 |
| MCF 7 (breast cancer) | 91.5 |

## Claims

1. A therapeutic composition for use in reducing cellular proliferation of malignant cells in a patient, the composition comprising a cell permeable peptide molecule comprising the amino acid sequence LGIPEAEY.

2. The composition of claim 1 for the use of claim 1, wherein the peptide molecule further comprises a cell permeable factor.

3. The composition of claim 2 for the use of claim 1,wherein said cell permeable factor is a cell penetrating peptide selected from RRRRRRR, RRRRRRRR, RRRRRRRRR, RRRRRRRRRR, RRRRRRRRRRR, RQIKIWFQNRRMKWKK, GRKKRRQRRRPPQ, GWTLNSAGYLLGKINLKALAALAKKIL, or GRKKRRQRRR.

4. The composition of claim 3 for the use of claim 1, wherein the cell penetrating peptide is covalently linked or conjugated to the peptide molecule; or recombinantly fused with the peptide molecule.

5. The composition of claim 3 for the use of claim 1, wherein the cell penetrating peptide further comprises a spacer or a linker sequence.

6. The composition of claim 1 for the use of claim 1, wherein the peptide molecule further comprises a nuclear localization sequence.

7. The composition of claim 1 for the use of claim 1, wherein the peptide is a retro-inverso isomer.

8. The composition of claim 1 for the use of claim 1, further comprising a chemotherapeutic agent.

9. The composition of claim 8 for the use of claim 1, wherein the chemotherapeutic agent is a DNA damaging agent selected from doxorubicin, irinotecan, cyclophosphamide, chlorambucil, melphalan, methotrexate, cytarabine, fludarabine, 6-mercaptopurine, 5- fluorouracil, capecytabine, cisplatin, carboplatin, oxaliplatin, or a combination thereof.

10. The composition of claim 1 for the use of claim 1, wherein the composition comprises a cell surface targeting factor selected from HER2, estrogen receptor, progesterone receptor, or EGFR.

11. The composition according to any of the preceding claims for the use of claim 1, in a method of treatment for reducing cellular proliferation of malignant cells comprising administering a therapeutically effective amount of the composition to contact the malignant cells.

12. The composition according to claim 11 for the use of claim 1, which method of treatment for reducing cellular proliferation further comprises administering radiotherapy.

13. The composition according to any of claims 1-10 for the use of claim 1 in sensitizing or augmenting cancer cells for cancer therapy comprising administering a therapeutically effective amount of the composition and a chemotherapeutic agent or radiotherapy.

14. The composition according to any of claims 1-10 for the use of claim 1 as a medicament for chemoprevention of cancer to prevent the transformation of the cells that express the cancer specific proliferating cell nuclear antigen (caPCNA) isoform to a malignant state or develop tumors.

15. The composition according to claim 14 for the use of claim 1, wherein the chemopreventative agent is administered to an individual with pre-cancerous cells.

## Patentansprüche

1. Eine therapeutische Zusammensetzung zur Verwendung beim Reduzieren einer Zellvermehrung maligner Zellen in einem Patienten, wobei die Zusammensetzung ein zellpermeables Peptid-Molekül aufweist, das die Aminosäuresequenz LGIPEAEY aufweist.

2. Die Zusammensetzung gemäß Anspruch 1 zur Verwendung gemäß Anspruch 1, bei der das Peptid-Molekül ferner einen zellpermeablen Faktor aufweist.

3. Die Zusammensetzung gemäß Anspruch 2 zur Verwendung gemäß Anspruch 1, bei der der zellpermeable Faktor ein zelldurchdringendes Peptid ist, das ausgewählt ist aus RRRRRRR, RRRRRRRR, RRRRRRRRR, RRRRRRRRRR, RRRRRRRRRRR, RQIKIWFQNRRMKWKK, GRKKRRQRRRPPQ, GWTLNSAGYLLGKINLKALAALAKKIL oder GRKKRRQRRR.

4. Die Zusammensetzung gemäß Anspruch 3 zur Verwendung gemäß Anspruch 1, bei der das zelldurchdringende Peptid kovalent an das Peptid-Molekül gebunden oder mit demselben konjugiert ist; oder mit dem Peptid-Molekül rekombinant verschmolzen ist.

5. Die Zusammensetzung gemäß Anspruch 3 zur Verwendung gemäß Anspruch 1, bei der das zelldurchdringende Peptid ferner einen Abstandshalter oder eine Binder-Sequenz aufweist.

6. Die Zusammensetzung gemäß Anspruch 1 zur Verwendung gemäß Anspruch 1, bei der das Peptid-Molekül ferner eine Kernlokalisierungssequenz aufweist.

7. Die Zusammensetzung gemäß Anspruch 1 zur Verwendung gemäß Anspruch 1, bei der das Peptid ein Retro-Inverso-Isomer ist.

8. Die Zusammensetzung gemäß Anspruch 1 zur Verwendung gemäß Anspruch 1, die ferner ein chemotherapeutisches Mittel aufweist.

9. Die Zusammensetzung gemäß Anspruch 8 zur Verwendung gemäß Anspruch 1, bei der das chemotherapeutische Mittel ein DNA-Beschädigungsmittel ist, das ausgewählt ist aus Doxorubicin, Irinotecan, Cyclophosphamid, Chlorambucil, Melphalan, Methotrexat, Cytarabin, Fludarabin, 6-Mercaptopurin, 5-Fluorouracil, Capecytabin, Cisplatin, Carboplatin, Oxaliplatin oder einer Kombination hieraus.

10. Die Zusammensetzung gemäß Anspruch 1 zur Verwendung gemäß Anspruch 1, wobei die Zusammensetzung einen Zelloberflächenzielfaktor aufweist, der ausgewählt ist aus HER2, einem Östrogenrezeptor, Progesteronrezeptor oder EGFR.

11. Die Zusammensetzung gemäß einem der vorherigen Ansprüche zur Verwendung gemäß Anspruch 1 in einem Verfahren einer Behandlung zum Reduzieren einer Zellvermehrung maligner Zellen, das ein Verabreichen einer therapeutisch wirksamen Menge der Zusammensetzung, um einen Kontakt zu den malignen Zellen herzustellen, aufweist.

12. Die Zusammensetzung gemäß Anspruch 11 zur Verwendung gemäß Anspruch 1, wobei das Verfahren der Behandlung zum Reduzieren einer Zellvermehrung ferner ein Verabreichen einer Strahlentherapie aufweist.

13. Die Zusammensetzung gemäß einem der Ansprüche 1 bis 10 zur Verwendung gemäß Anspruch 1 beim Sensibilisieren oder Verstärken von Krebszellen zur Krebstherapie mit Verabreichen einer therapeutisch wirksamen Menge der Zusammensetzung und eines chemotherapeutischen Mittels oder Strahlentherapie.

14. Die Zusammensetzung gemäß einem der Ansprüche 1 bis 10 zur Verwendung gemäß Anspruch 1 als ein Medikament zur Chemoprävention von Krebs, um die Umwandlung der Zellen, die die krebsspezifische sich vermehrende Zellkern-Antigen- (caPCNA-) Isoform ausdrücken, in einen malignen Zustand oder die Entwicklung von Tumoren zu verhindern.

15. Die Zusammensetzung gemäß Anspruch 14 zur Verwendung gemäß Anspruch 1, bei der das chemopräventive Mittel einem Menschen mit Krebsvorstufenzellen verabreicht wird.

## Revendications

1. Composition thérapeutique utilisée pour réduire la prolifération cellulaire de cellules malignes chez un patient, la composition comprenant une molécule peptide à perméabilité cellulaire comprenant la séquence d'acides aminés LGIPEAEY.

2. Composition selon la revendication 1 utilisée selon la revendication 1, dans laquelle la molécule peptide comprend en outre un facteur de perméabilité cellulaire.

3. Composition selon la revendication 2 utilisée selon la revendication 1, dans laquelle ledit facteur de perméabilité cellulaire est un peptide pénétrateur de cellules sélectionné parmi RRRRRRR, RRRRRRRR, RRRRRRRRR, RRRRRRRRRR, RRRRRRRRRRR, RQIKIWFQNRRMKWKK, GRKKRRQRRRPPQ, GWTLNSAGYLLGKINLKALAALAKKIL, ou GRKKRRQRRR.

4. Composition selon la revendication 3 utilisée selon la revendication 1, dans laquelle le peptide pénétrateur de cellules est lié ou conjugué de façon covalente à la molécule peptide ; ou fusionnée de façon recombinante avec la molécule peptide.

5. Composition selon la revendication 3 utilisée selon la revendication 1, dans laquelle le peptide pénétrateur de cellules comprend en outre une séquence espaceur ou de liaison.

6. Composition selon la revendication 1 utilisée selon la revendication 1, dans laquelle la molécule peptide comprend en outre une séquence de localisation nucléaire.

7. Composition selon la revendication 1 utilisée selon la revendication 1, dans laquelle le peptide est un isomère rétro-inverso.

8. Composition selon la revendication 1 utilisée selon la revendication 1, comprenant en outre un agent chimiothérapique.

9. Composition selon la revendication 8 utilisée selon la revendication 1, dans laquelle l'agent chimiothérapique est un agent d'altération de l'ADN sélectionné parmi la doxorubicine, l'irinotécan, la cyclophosphamide, le chlorambucil, le melphalan, le méthotrexate, la cytarabine, la fludarabine, la 6-mercaptopurine, le 5-fluorouracil, la capécitabine, le cisplatin, le carboplatine, l'oxaliplatine, ou une combinaison de ceux-ci.

10. Composition selon la revendication 1 utilisée selon la revendication 1, dans laquelle la composition comprend un facteur de ciblage de surface de cellule sélectionné parmi un HER2, un récepteur d'oestrogène, un récepteur de progestérone, ou un EGFR.

11. Composition selon une quelconque des revendications précédentes utilisée selon la revendication 1, dans un procédé de traitement pour réduire la prolifération cellulaire de cellules malignes comprenant l'administration d'une quantité thérapeutiquement efficace de la composition pour entrer en contact avec les cellules malignes.

12. Composition selon la revendication 11 utilisée selon la revendication 1, dont le procédé de traitement pour réduire la prolifération cellulaire comprend en outre l'administration de radiothérapie.

13. Composition selon une quelconque des revendications 1 à 10 utilisée selon la revendication 1, dans la sensibilisation ou l'augmentation de cellules cancéreuses pour la thérapie du cancer comprenant l'administration d'une quantité thérapeutiquement efficace de la composition et un agent chimiothérapique ou de la radiothérapie.

14. Composition selon une quelconque des revendications 1 à 10 utilisée selon la revendication 1 comme médicament pour la chimioprévention du cancer afin d'empêcher la transformation des cellules exprimant un isoforme spécifique de cancer d'antigène nucléaire de cellules proliférantes (caPCNA) en un état malin ou le développement de tumeurs.

15. Composition selon la revendication 14 utilisée selon la revendication 1, dans laquelle l'agent chimiopréventif est administré à un individu souffrant de cellules précancéreuses.
